# EUROPEAN PATENT SPECIFICATION

(11) **EP 2 847 369 B1**
(45) Date of publication and mention of the grant of the patent: **11.07.2018**
(21) Application number: 13732807.6
(22) Date of filing: 10.05.2013
(51) Int. Cl.: D01F 9/00, D01D 5/40, D01D 5/06

(54) **METHOD OF PREPARATION OF POLYSACCHARIDE MICROFIBRES, WOUND COVERS COMPRISING THE OBTAINED MICROFIBRES, METHOD FOR PRODUCING THE WOUND COVERS**
VERFAHREN ZUR HERSTELLUNG VON POLYSACCHARIDMIKROFASERN, WUNDVERBÄNDE MIT DADURCH ERHALTENEN MIKROFASERN, VERFAHREN ZUR HERSTELLUNG DER WUNDVERBÄNDE
PROCÉDÉ DE PRÉPARATION DE MICROFIBRES DE POLYSACCHARIDE, PANSEMENTS COMPRENANT LES MICROFIBRES OBTENUES, PROCÉDÉ DE PRÉPARATION DES PANSEMENTS

(30) Priority: 11.05.2012 CZ 20120306
(43) Date of publication of application: 18.03.2015
(73) Proprietor: Contipro a.s., 56102 Dolní Dobrouc (CZ)
(72) Inventor: BURGERT, Ladislav, 530 02 Pardubice (CZ); HRDINA, Radim, 530 03 Pardubice (CZ); VELEBNY, Vladimir, 564 01 Zamberk (CZ); ABDEL-LATTIF, Abdel Mohsen, Disia Village Fayoum City (EG); SULAKOVA, Romana, 562 01 Usti nad Orlici (CZ); SOBOTKA, Lubos, 500 09 Hradec Kralove (CZ); BETAK, Jiri, 143 00 Praha 4 - Modrany (CZ); SMIRNOU, Dzianis, 562 01 Usti nad Orlici (CZ)
(74) Representative: Dvorakova, Martina
(86) International application number: PCT/CZ2013/000063
(87) International publication number: WO 2013/167098

(56) References cited:
- WO-A1-2013/056312
- WO-A2-2006/026104
- CZ-B6- 302 994
- US-A- 4 205 025
- US-A1- 2003 163 073
- US-A1- 2003 205 839
- US-A1- 2010 247 908
- RUPPRECHT A: "Wet Spinning of Hyaluronic Acid. Preparation of Oriented Samples", ACTA CHEMICA SCANDINAVICA.SERIES B - ORGANIC CHEMISTRY AND BIOCHEMISTRY, MUNKSGAARD. COPENHAGEN, DK, vol. B33, no. 10, 1 January 1979 (1979-01-01), pages 779-780, XP002483473, ISSN: 0302-4369

## Description

### Field of the invention

This invention relates to a method for preparing polysaccharide staple microfibres, wound covers based on the microfibres, and the method of their preparation.

### Background of the invention

Treatment of skin defects, and acute, or chronic wounds is a very important part of the modern medicine. This also relates to the increasing age of population being treated with the use of more and more modern procedures. Wound healing is a very complicated process which is nowadays studied intensively. Even though the partial steps of the healing process are known, the whole complex is still undiscovered. Nowadays the wet healing is preferred, based on supplying the humidity from the dressing, and using the regulated drainage of the tissue fluid. Use of a vacuum pump with the low pressure is also modern. During the last 10 years, the system based on the utilization of biological and physical properties of the hyaluronic acid has been prepared. Especially in combination with an appropriate antimicrobial agent, its action in the location of the healing wound is ensured. So far, experience with this therapy has been positive, and the advantageousness of the antiadhesive, hydrophilic, and healing properties of the hyaluronan-iodine complex has been proved in many papers.

The wound covers can be further improved by utilization of biopolymers spinning. With the use of this method, a large surface acting on the contact wound surface, better contacting of the cells taking part in the healing with the active substances bound to the fibres, the dressing structure improving the properties of the extracellular matrix of the tissue being healed, and the right circulation of the tissue fluid will be ensured.

The staple fibres can be processed into the so-called staple yarns or they can be successfully processed into nonwoven textiles. The nonwoven textiles usually present a continuous layer of staple fibres where the primary fibres are arranged randomly. In contrast to the conventional textiles (prepared by weaving, knitting, crocheting), the unwoven textile is based on a web of these randomly arranged fibres which can be both short and long. This layer-forming nonwoven textile can be compact and paper-like, or it can represent a porous and "fluffy" structure. The layer thickness (nonwoven textile) is usually from 25 µm to several centimeters. The layer weight is therefore from about 5 g/m² to 1 kg/m². The mechanical properties (nonwoven textile), such as the tear strength, bending strength, and the like, are given by the primary fibres strength, and also by adhesive and physical and chemical forces between the individual fibres, which are crossed randomly (Encyclopedia of Textiles, Fibers, and Nonwoven Fabrics, Encyclopedia Reprint Series, Ed. Martin Grayson, John Wiley & Sons, Inc., 1984).

As regards the fibres themselves, they can be classified as conventional fibres, microfibres and nanofibres. The microfibres are defined as the fibres with the sheerness bellow 1 dtex, i.e. the weight of a fibre of 1 000 m long is below 0,1 g. The nanofibres are characterized with their diameter of units to hundreds nanometers, due to their character their sheerness in decitexes is not determined. Unwoven textiles can be prepared from all types of these fibres. As regards the chemical nature of the primary fibres, they can be both natural and synthetic fibres. It is obvious that an unwoven textile can be prepared by mechanical mixing of various fibres of different sheerness or chemical nature. It is also possible to combine a conventional textile (woven textile, knitted textile, and the like) with a nonwoven textile, i.e. a layer of the nonwoven textile is "applied" on the conventional textile. These combinations are used in the cases where e.g. enhancement of the mechanical properties of the final product is demanded, or a "multi-layer" material is desired, or a combination of polymers, where a final product has a particular function, for example it stimulates wound healing and the like, is concerned. The reason for "mixing" various polymers (fibres) can be also economical (a combination of expensive and cheap fibres), especially in case of the so-called functional textiles.

Interestingly, in the past the term microfibres was related to the synthetic fibres, especially polyester, and polyamide. The techniques of producing staple fibres from synthetic polymer melt can be found e.g. in the publication Nakajima T, Kajiwara K, McIntyre J E, 1994. Advanced fibre Spinning Technology. Woodhead Publishing.

Microfibres or nanofibres can be, in general, prepared from a polymer solution by various techniques. Probably the most sophisticated one is the so-called electrospinning, or spinning in a strong electrical field (5 to 50 kV), where a polymer solution (or a melt, in case of synthetic polymers) is injected via a thin nozzle (needle) into electrostatic field, where the nozzle and the collector are connected to a voltage source. A polymer droplet flowing out of the nozzle is stretched by this field, and that leads to the production of a nano- or microfibre, with a solvent evaporating at the same time. We can say that the number of publications discussing the electrospinning increases exponentially during the time (D. Li, Y. Xia. Electrospinning of Nanofibres: Reinventing the Wheel?. Advanced Materials. 2004, 16, 1151-1170).

The microfibres from, in general, biopolymers, can be obtained also by a strong mechanical action on a "raw" starting natural material. For example, the microfibrillated cellulose (MFC) can be prepared by microfluidization technique from a wood substance (the preparation of MFC is described for example in J. Y. Zhu et al, Green Chem., 2011, 13, 1339).

Another method to obtain the microfibres from biopolymers (sodium alginate, starch,keratin) is described in the publications from Polish authors (Dariusz Wawro a kol., Microfibrids from Natural Polymers', Fibres & Textiles in Eastern Europe, v. 10, No. 3/2002 (38), pp. 23-26, 2002), where the principle lies in the use of non-stationary coagulation bath. There is a principle of a stream of a polymer solution coming out of a nozzle being driven by a stream, preferably perpendicular, of the coagulation bath. This leads to the fibrillation and microfibres formation. However, there is a disadvantage of the fibres prepared by this process being very short, according to the said document their length is maximum 800 µm, so they form a hardly processable pulp. The same author described the preparation of chitosan microfibres by the same method (Dariusz Wawro a kol., Fibres & Textiles in Eastern Europe (2006), 14(3), 97-101.).

The use of nonstationary coagulation bath is described in the U.S. Patent No. 5868973 (1999) 'Process and apparatus for producing fibres from cellulose derivatives'. The prepared microfibres are also very short.

Hyaluronic acid (A) has a chemical structure of a linear polyelectrolyte composed of repeatedly altering units of β-(1,3)-D-glucuronic acid and β-(1,4)-N-acetyl-D-glukosamine repeating continually and creating (-4GlcUAβ1-3GlcNAcβ1-)ₙ long chains. Every repeating unit has one carboxyl group, four hydroxyl groups, and an acetamide group.

| | |
|---|---|
| 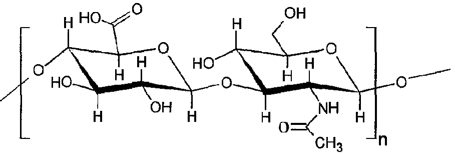 | A |

Primary and secondary hydroxyl groups are moderately acidic, and they can be ionized by alkali ionization, for example by sodium hydroxide, where, however, pKₐ is above the value of 14. The carboxyl group belongs to a group of medium acids neutralized by alkali into salts, hylauronates, e.g. sodium hyaluronate. The mixture of a salt and a free acid is referred to as hyaluronan. For example, pKₐ of the free form of hyaluronic acid in water varies around 3.45, in 0.2M NaCl it is 2.95 (L. Lapč̌ík et al.: Chemické listy 85, 281-298, 1991). Hyaluronic acid is characterized by a high molecular weight of 5.10⁴ to 5.10⁶ g.mol⁻¹, depending on the source from which it has been obtained. This polysaccharide is water soluble within the whole pH range, in the form of a salt. The hyaluronic acid is a unique biopolymer intended for therapeutic applications acknowledged as a versatile surface material improving the biocompatibility of remedies. A review can be found for example in the publication (S. Dumitriu, Polysaccharides: Structural diversity and functional versatility, Marcel Dekker Inc. 1998, ISBN 0824701275).

Hyaluronic acid, more specifically hylauronan, has a beneficial effect on wound healing. This is due to its physical, chemical, and biological properties. The polymer is a strong hydrophile, ensuring therefore a good transportation of a tissue fluid, and favourable rheological properties in the location of the healing wound, it prevents its drying, and simultaneously prevents serious adhesion of the bandage to the wound. The biological properties of hyaluronan are associated with its influence on inflammation processes, new vascular tubes formation, bonding on lymphatic vessels, and cell receptors (CD 44 receptors) stimulation. All these effects improve healing of wounds and skin defects (Sobotka L, Smahelova A, Pastorova J, Kusalova M.: A case report of the treatment of diabetic foot ulcers using a sodium hyaluronate and iodine complex*.*

Int J Low Extrem Wounds. 2007 Sep;6(3):143-7. And Cutting KF.: Wound healing through synergy of hyaluronan and an iodine complex. J Wound Care. 2011 Sep; 20(9):424, 426, 428-30).

Chitin (B1) is the second most common polysaccharide found in the nature. It consists of repeating *β*-(1,4)-*N*-acetyl glukosamine units. It is water insoluble, soluble in organic solvents such as hexafluoro isopropanole, hexafluro acetone, chlorinated alcohols conjugated with aqueous solutions of mineral acids, and *N,N*-dimethylacetamide containing 5 % of lithium salts (M. N. V. Ravi Kumar, Reactive and Functional Polymers 46 (2000) 1-27).

Chitosan (B2) is a form of chitin, where at least 50 % of *β*-(1,4)-*N*-acetyl glukosamine units are deacetylated. Its properties are influenced by the degree of deacetylation (ratio of acetylated to deacetylated units). This cationic polysaccharide is water soluble at acidic pH levels lower than pKa of chitosan (pKₐ 6.5). Chitin and chitosan are commercially obtained from seashells (crabs, lobsters, crayfish), or by extraction of the cell walls of certain fungi.

Both these polysaccharides are of wide industrial use, for example in water treatment, in cosmetics as an additive to soaps and shampoos, or in medicine for preparation of bandages intended to ensure wound hydration in the so-called wet healing (Dai T, Tanaka M, Huang YY, Hamblin MR: Chitosan preparations for wounds and burns: antimicrobial and wound-healing effects. Expert Rev Anti Infect Ther. 2011, Jul; 9(7):857-79). Chitin and chitosan are also used for production of skin substitutes (Tseng HJ, Tsou TL, Wang HJ, Hsu SH.: Characterization of chitosan-gelatin scaffolds for dermal tissue engineering. J Tissue Eng Regen Med. 2011 Oct 28. doi: 10.1002).

The said polysaccharides have substantial sequestration effects (they are capable to bond metal ions). Chitosan is renewable and biodegradable, and at the same time it has also an antibacterial effect (N. Liu et al., Carbohydrate Polymers 2004; 64; 60-65). Its salts with organic (lactate, acetate, glycolate, succinate) and mineral acids (hydrochloride) are water soluble, and at the same time they have haemostatic effect, which can be also related to the ability of bonding haemocoagulative molecules.

| | |
|---|---|
| 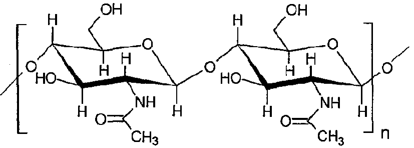 | B1 |
| 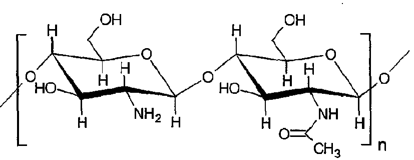 | B2 |

Chitin/chitosan-glucan complex is obtained from the mycelium of certain fungi species (*Aspergillus sp., Penicillium sp., Schizophyllum commune*). It is a mixture of chitin/chitosan and *β*-(1,3)-D-glucan which are joined with a glycosidic bond. The weight ratio of chitin/chitosan and glucan is from 0.001:99.99 to 99.99:0.01. This polysaccharide can be in the form of a pharmacologically acceptable salt - lactate, glycolate, acetate, succinate, hydrochloride.

Chitin/chitosan-glucan complex can have a beneficial effect on healing due to an immuno-modulative effect of glucan. In addition, it can entrap humidity in the defect area, and therefore ensure the so-called wet healing.

Schizophylan (D) is an extracellular polysaccharide produced by the fungi *Schizophyllan commune* ATCC 38548. This neutral polysaccharide consists of the chain of *β*-(1,3)-D-glucopyranose units, where *β*-(1,6)-D-glucopyranose is bonded on every third *β*-(1,3)-D-glucopyranose unit, and the molecule forms a triple helix. Its molecular weight varies within the range of 6-12x10⁶ g.mol⁻¹. In dimethylsulfoxide, at the temperatures above 120 °C or pH above 12, untwisting of triple helix occurs and the molecule is then formed by single chains, which leads to a decrease of the molecular weight by 1/3 (U. Rau, Methods in Biotechnology, 1999, Volume 10, 43-55, DOI: 10.1007/978-1-59259-261-6_4). This polysaccharide is degraded in a mammal body, and it has many remedial and immunomodulative effects (Wakshull, Immunopharmacology 41 (1999) 89-107).

| | |
|---|---|
| 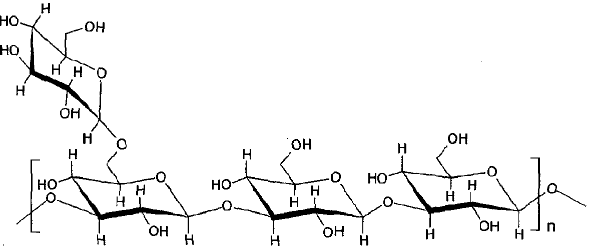 | D |

Xanthan (E) is a water-soluble polysaccharide produced by Xanthomonas campestris bacteria, and the most frequently it is used as a food additive (Davidson, Robert L. (1980). Handbook of Water-soluble Gums and Resins. McGraw Hill. ISBN 0070154716).

| | |
|---|---|
| 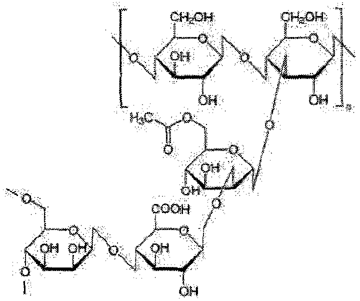 | E |

Alginate (F), or the alginic acid, is also a water-soluble anionic polysaccharide which strongly absorbs moisture. The production thereof can be found in (Remminghorst and Rehm (2009). "Microbial Production of Alginate: Biosynthesis and Applications". Microbial Production of Biopolymers and Polymer Precursors. Caister Academic Press. ISBN 978-1-904455-36-3). Because of its capability of water entrapping in the location of a wound, alginate is widely used in the cases of the need of wound moisturizing and ensuring the decomposition and gradual dissolving of necrotic tissues. It is often combined with antimicrobial agents.

| | |
|---|---|
| 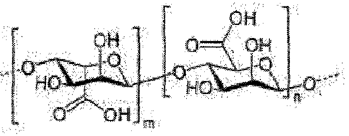 | F |

Oxycellulose is a cellulose derivative where a part of -CH₂-OH groups is oxidized into -COOH groups. Oxycellulose water-solubility depends on the content of carboxyl groups. The solubility of almost 100 % is achieved when the -COOH groups content is above 20 %. Desired haemostatic effects are achieved within the range of 16 to 19 % of COOH.

Carboxymethyl cellulose (G) is a synthetic cellulose derivative, and it is an anionic polysaccharide, often used in the form of a sodium salt which is water soluble. It is also a food additive (Sodium carboxymethyl cellulose (Cellulose gum). *Codex Alimentarius.* 2009).

| | |
|---|---|
| 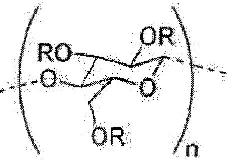 | G |
| R = H, CH₂COOH | |

Many biopolymers have the disadvantage of non-fusibility (their melting point is above their decomposition temperature). For spinning, especially for the purpose of nonwoven textiles production, it is not possible to use progressive technologies of fibre production, such as "spunbond" (i.e. direct application of a fibre formed form a melt by a nozzle onto an endless belt where a web layer - the base of the nonwoven textile - is formed), or "meltblown" (i.e. the polymer melt leaving the nozzle is entrained with a hot air flow, which helps to form a characteristic type of the fibre as a base of a nonwoven textile).

As the polysaccharides do not flux, the method called wet spinning is used for preparing the fibres. A polysaccharide dissolved in an appropriate solvent is extruded through various types of nozzles (circular or slot nozzle, or a nozzle with various profiles - radial, spherical triangle, and the like) directly into a non-solvent (precipitation bath), where a fibre or a film is formed. The description of the wet spinning method can be found for example in T.P. Nevell, S.H. Zeronian: Cellulose Chemistry and its Applications. John Wiley & Sons 1985, p. 455 - 479. Or: J.E. McIntyre: The Chemistry of Fibres. Edward Arnold, London 1971, p.15.

The wet spinning method can be modified into the so-called dry-wet spinning. It is used, for example, for spinning of certain types of aromatic polyamides or fibres from regenerated cellulose of the Tencell, Lyocell types, and the like. The principle of dry-wet spinning consists in that a stream of a polymer solution leaving the nozzle passes through an air gap before its entering into the precipitation bath, this gap has a length from a few millimeters to a few centimeters (this technique is described e.g. in documents WO 2000/063470, DE 10331342, JP 2008208480). The dry-wet spinning technique has been originally used for spinning of hyaluronan in the document (Buřgert L., et al., Hyaluronová vlákna, způsob jejich přípravy a použití, PV 2010-1001).

There are known methods of spinning of hyaluronic acid into a fibre, for example A. Rupprecht (Acta Chem. Scand., B33, No. 10, 1979) spun the potassium salt of hyaluronic acid in 75 - 80 % ethanol comprising 0.1M KCl, he coiled the fibres into a disc, so the fibres aglutinated by drying into a film comprising a "spatially orientated" hyaluronic acid. This way of spinning naturally did not lead to fibres processable in the textile industry.

Further, E.D.T. Atkins et al. (Biochem. J. 128, 1255-1263, 1972) prepared model fibres from hyaluronic acid and sodium hyaluronate in such a way that first they prepared a film which they cut into thin strips. For these "model fibres" cut from films (and drawn) they determined a crystal modification with the use of X-ray structural analysis, including the dimensions of an elementary crystal cell. They found out that the elementary cell of a non-drawn sodium hyaluronate fibre was of a hexagonal crystal system and the macromolecules were arranged antiparallely supposing the hydrogen bonds between amide groups of acetylated glucosamine. Base dimension: a = 1.17 nm (or 2.02 nm). Macromolecule profile diameter approx. 0.673 nm. Dimension c of the elementary cell (along the fibre axis) was 2.85 nm.

They have also found that conditioning of sodium hyaluronate based fibres at 60 °C and relative humidity of 75 % lead to the formation of a new hexagonal crystal lattice, a = 1.87 nm, maintaining c = 2.85 nm. The change of the lattice type (with maintaining the crystal modification) is also known in cases of other fibres - polymers - e.g. cellulose in the cotton fibre.

The same author determined the monoclinic crystal system with the elementary cell dimension of a = 1.96 nm (but it is not fully defined) at the fibres prepared from hyaluronic acid (acid form).

The document WO 2009/050389 describes the preparation of a yarn with the use of wet spinning method, where this yarn comprises hyaluronic acid in the acid form, acetic acid, and it is partially water soluble. The preparation of a fibre consists of the following steps: a) preparation of an aqueous solution of hyaluronic acid, or a salt thereof, preferably sodium salt, where the hyaluronic acid concentration is higher than 0.8 %, preferably within the range of 1 to 2 % by weight; b) extrusion of the solution through the nozzle into a spinning bath; c) forming the fibre in the bath, which consists of acetic acid and water, where the acetic acid concentration is higher than 80 %, preferably higher than 90 %, the most preferably 99 %. The obtained yarn has a diameter higher than 100 µm. The authors of this document declare that the fibres prepared by their procedure can be used for both the woven and nonwoven textiles. In fact, due to their small strength (according to the comparative example 10, the tearing occurs at the stress of 67 MPa), the fibres are not applicable for the preparation of woven fabric or knitted fabric, so they are applicable for preparation of nonwoven textiles only. The strengths of textile fibres produced in a conventional manner vary within the following intervals: cellulose fibres 1.8 to 2.7 cN.dtext⁻¹; synthetic fibres above 4 cN.dtext⁻¹; coefficient of variation up to 10 %. Loop (knot) strength of the fibre should be approximately 80 % of the fibre strength without deformation. It applies especially to weaving, knitting, and bobbin lace work. For non-woven textiles, the loop strength of the fibre is not essential.

The above mentioned document PV 2010-1001 describes the preparation of fibres comprising the hyaluronic acid characterized in that the fibre consists of hyaluronic acid and/or at least one compound comprising hyaluronic acid and metal ions, wherein the fibre can optionally comprise metal salts originating from the production process, having the molecular weight of macromolecules within the range of 100 kDa to 3 MDa. The prepared fibres are characterized by that the fibre (monophile, primary fibre) diameter is 4 µm to 1 mm, length weight 0.1 to 30 g/1000 m (0.1 to 30 tex), tensile strength 0.5 to 3 cN.dtex-1, and loop strength 20 to 80 % of tensile strength. The solution is characterized also by the preparation of silk tow comprising 2 to 50 primary fibres. Therefore it concerns the fibres with properties useful for the production of both woven and nonwoven textiles.

The preparation of chitin fibres via the wet spinning method from chitin solution is described for example in the U.S. Patent 1029727 (1977), where chitosan is dissolved in trichloroacetic acid, and the fibres of high strength are prepared by the wet spinning method.

The Japanese document (OPI) No. 127500/1978 describes a similar method differing only in that chitin is dissolved in dichloroacetic acid. The prepared fibre has a lower strength than when using the method employing trichloroacetic acid.

The U.S. Patent 3,988,411 (1976) uses hexafluoro isopropyl alcohol or hexafluoro acetone sesquihydrate to dissolve chitin. Acetone is used as the coagulation bath.

The U.S. Patent 4,029,727 (1977) uses mixtures of trichloroacetic acid or dichloroacetic acid, chloral hydrate, and halogenated hydrocarbon (e.g. CCl₄) to dissolve chitin. Acetone is used as the coagulation bath, then the fibres are neutralized with alkalis, washed with 2-propanol, and dried.

The U.S. Patent 4,431,601 (1984) uses mixtures of trichloroacetic acid and halogenated hydrocarbon (e.g. CCl₄) to dissolve chitin. Acetone is used as the first coagulation bath, and methanol is used as the second coagulation bath. The produced fibres are useful as surgical sutures. The U.S. Patent US 4,932,404 (1990) describes a very similar method.

The U.S. Patent 4,857,403 describes the preparation of tough fibres of "chitin acetate/formate" and "chitosan acetate/formate". In fact, it concerns a method where the derivatives of chitin and chitosan are prepared first (by *O*-acylation and *N*-acylation), and then they are spun by a conventional method. The problem of this patent is that the procedure with chitosan comprises dissolving chitosan (40 g) in formic acid, then adding the ice cold acetic acid, and anhydride (500 ml). The mixture is agitated for 12 hours at 0 °C. At these given conditions, the rate of acetylation of chitosan amide groups, and therefore the conversion of chitosan to chitin, is questionable. *N-*acetyl and *O*-acetyl chitin and chitosin derivatives are herein called acetates. Another problem, naturally, is that the amine group protonizes by acid treatment to give the respective salts (-NH₂ + CH₃COOH → -NH₃^{+ -}OOCCH₃). In the end, the resulting fibres are *O*-acyled and *N*-acyled chitine and chitosane derivatives. The following U.S. Patent 4,861,527 (1989) tries to solve this problem relating to chitosan in that the fibers prepared by the methods of the previous patent are immersed into the concentrated aqueous solution of NaOH (50-70 % by weight of NaOH). This induces *O*- and *N*- deacetylation, and also dissapearing of the amide groups and re-creation of -NH₂ groups. However, the problem is that chitosan in its neutral form (amine groups are not protonized) does not have haemostatic and antimicrobial effects. Of course, it has a strong chelating effect.

The preparation of chitosan fibres by the wet spinning method from chitosan solution is described for example in the document (OPI) No. 112937/1981, where chitosan is dissolved in water at acidic conditions, the solution is filtrated and spun into the coagulation bath comprising anion surfactants.

The document (OPI) No. 116418/1984 describes dissolving of chitosan in water acidified with dichloroacetic acid. Further, this document describes chelation of metal ions by prepared fibres.

The U.S. Patent 4,464,321 (1984) describes a method of preparation of chitosan hollow fibres designed for ultrafiltrations, where an acidic chitosan solution is spun into an alkaline coagulation bath comprising a plastificator (glycerin). The interesting fact in this method is that the stream leaving the nozzle (designed for forming hollow fibres) passes through an ammonium atmosphere before its entrance into the coagulation bath. Therefore, it concerns a wet-dry spinning method.

The document (OPI) No. 106901/1988 describes a method, where chitosan is dissolved at 40 °C in water acidified by acetic acid, and thus the solution comprising 3 % of chitosan and 0,5 % of acetic acid is prepared. The solution is spun at 30 °C in the coagulation bath comprising aqueous 1% NaOH. The formed fibres are washed and dried. The fibre tensile strength is 2.44 g/denier, elongation at break 10.8 %, and knot strength 1.77 SG/denier.

In the publication (Shigehiro Hirano a kol., Carbohydrate Polymers 38 (1999) 293-298) the preparation of staple chitosan fibres, which are in addition plastified by ethylene glycol is described.

The essential problem of pure chitosan consists in that, according to our experiences, these fibres have a small strength and are fragile. Another problem is that chitosan acidic aqueous solutions are logically spun into an alkaline solution thereby the chitosan has no protonized amino groups and therefore it loses its haemostatic and antibacterial effect (Fouda, M.M.G. et al., Use of dressing. International Journal of Diabetes Mellitus. 2009, 1, 61-64).

We can conclude that wound covers based on staple microfibres from hyaluronic acid and/or hyaluronan, with the fibres long enough (more than 800 µm) to prepare tough nonwoven textile with a very low surface weight (below 5 g/m²), cotton wool, tampons, or material usable for spinning, have not been known so far. If a spinning nozzle and a draw-off (and curling) apparatus is arranged properly, it would be possible to process the forming staple microfibres into staple yarns.

We can also conclude that neither wound covers based on staple microfibres longer than 800 µm made from pure schizophylan, chitin, chitin/chitosan-glucane complex, internal mixture of chitin and chitosan, or water soluble chitosan salt, are known, even if we take into account the fact that chitin and also chitosan fibres exist and it is not possible to distinguish exactly between chitin and chitosan. Herein, the internal mixture is understood as the individual chitin and chitosan molecules are "entwined" mutually.

Said drawback is eliminated by the present invention.

The document US 4,205,025 discloses a process for producing polysaccharide fibrids by extruding an aqueous solution of carboxymethyl cellulose into 80% isopropyl alcohol agitated with high shear, refining fibres in the blender, followed by the production of a sheet of paper from said fibrids. The composition of the coagulation bath and the gradual dilution thereof during the extrusion process leads to sticking the fibres together, making an inhomogeneous dispersion.

The document US 2010/247,908 discloses a method of production of nanofibers, including the steps of dissolving the polymer which is to be spun in a polymer solvent, introducing the polymer solution into a dispersion medium, whereby polymer solution droplets are formed, shearing the dispersion medium whereby the droplets elongate and stiffen, thus forming nanofibers. Examples only mention polystyrene, cellulose acetate and poly(L-lactic) acid as polymers, chloroform or a mixture of alcohols as the solvent, and glycerol + ethanol as the dispersion medium. The use of chloroform hinders the fibres to be used for medicinal purposes.

The patent application No. US 2003/163,073 discloses a method of preparation of polyelectrolyte solid material system in the form of flocks, flock composites, nonwovens, or paper, by dissolving an anionic polyelectrolyte in water, dissolving a cationic polyelectrolyte in water, combining the two solutions and passing the combined solution through a mixing tube into a hydrophilic precipitant solution. The list of suitable anionic polyelectrolytes consists of carboxymethyl cellulose, polyacrylic acid, polymethacrylic acid, pectin and carboxymethyl chitosan, and the only example of a cationic polyelectrolyte is chitosan. The disadvantage of said method is the use of acetone in the coagulation bath.

The patent application No. US 2003/205,839 relates to a process of making bioengineered collagen fibrils by wet spinning, i.e. extruding a material into a flow of coagulation agent and filtrating the fibres from the coagulation agent. The solvents for collagen are dilute acetic acid, hydrochloric acid, citric acid and formic acid, and the coagulation agent is PEG in phosphate buffer One of the drawbacks of said method is that the collagen fibres thereby produced always contain residual PEG, which, as a body-foreign substance, is undesirable for pharmaceutical or medicinal purposes.

### Summary of the Invention

The invention relates to the method of preparation of polysaccharide staple microfibres, where the spinning solution of a polysaccharide of the concentration of 0.01 to 0.8 % by weight is prepared, the polysaccharide having the molecular weight of 60 kDa to 3 MDa and being selected from the group comprising hyaluronic acid, a compound comprising hyaluronic acid and metal ions, schizophylan, chitin/chitosan-glucan complex, a compound comprising chitin/chitosan-glucan complex and metal ions, internal mixture of chitin and chitosan, a compound comprising internal mixture of chitin and metal ions, sodium alginate, potassium alginate, ammonium alginate, xanthan, xanthan sodium salt, xanthan potassium salt, oxycellulose, oxycellulose sodium salt, oxycellulose potassium salt, or mixtures of said polysaccharides, in an aqueous medium, which is spun in a non-stationary coagulation bath consisting of 97.5 to 100 % C₁-C₃ alcohol, wherein the coagulation bath is stirred at 2,000 to 2,500 rpm. The pH of the aqueous medium is preferably from 1 to 13. The spinning solution can pass through the air passage of the length of 1 to 200 mm before its entry into the coagulation bath. The spinning solution can also be stored for 5 to 24 hours in the refrigerator at the temperature of -40 °C to -10 °C before spinning. In a preferred embodiment, the spinning solution can comprise calcium or zinc salt in the concentration from 0.1 to 50 % by weight selected for example from the group comprising acetates, formates, chlorides, sulphates, fluorides, nitrates, and hydrates of these salts.

The temperature of the coagulation bath is preferably within the range of 18 to 30 °C. The fibres are transferred from the coagulation bath to a stationary maturation bath for 20 minutes to 96 hours, wherein the stationary maturation bath comprises C₁-C₃ alcohol, and can comprise also up to 10 % by weight of an organic acid.

In another preferred embodiment of the invention, the fibres are metalized for 20 minutes to 25 days in a metallization bath comprising C₁-C₃ alcohol or a mixture of C₁-C₃ alcohol and an organic acid, and a salt of a polyvalent metal selected from the group comprising calcium, magnesium, copper, manganese, zinc, chromium, iron, and cobalt. The fibres can also be shortened, in a shortening bath comprising C₁-C₃ alcohol or a mixture of C₁-C₃ alcohol and an organic acid, from the starting length of 0.8 to 3 cm to a length suitable for a method of preparation of a nonwoven textile.

A fibre suspension can preferably be homogenized in a homogenization bath comprising C₁-C₃ alcohol or a mixture of C₁-C₃ alcohol and an organic acid.

The metallization of fibres can be run directly in the coagulation bath, the maturation bath, the shortening bath, or the homogenization bath by adding a salt of a polyvalent metal, or the fibres are transferred to a new metallization bath. The shortening of fibres can be run directly in the maturation bath or the fibres are transferred to a new shortening bath. The homogenization of fibres can be run directly in the shortening bath or the fibres are transferred to a new homogenization bath.

Then the fibres are collected from the last bath and dried, wherein they can first be washed with alcohol before drying.

Further, the invention relates to the method of production of a cover based on polysaccharide fibres, where the fibres are prepared by the method disclosed above, or the mixture of fibres is prepared by the method disclosed above, or the mixture is prepared of fibres prepared by the method disclosed above and the fibres selected from the group comprising the fibres based on natural mycelium processed by a method maintaining the fibre character of natural mycelium, then the fibres are removed from the solution by filtration and the filtration cake is dried. The filtration cake can be compressed and/or washed with alcohol before drying. For example, in case of hyaluronan, only the "abundant water" is removed by alcohol, whereas the water bound in hyaluronan ensuring the bonds between the -COOH and -NHCOCH₃ groups is kept, so the hyaluronan is not denatured in an undesirable way. The fibres can be also filtered off onto a suitable pad, the filtration cake and the pad are compressed and then dried, and the pad can be optionally removed from the dried filtration cake.

H-bonds are established between the individual fibres by the "wet" compression of the filtration cake, so the nonwoven textile (a cover) is compact, tough and elastic. The same interactions are established also with the fibres from the more hydrophilic pad, for example polyamide.

After compression, the wet cover of the wound comprises above all the used alcohol, but due to a high hydrophility of, for example, hyaluronic acid, it comprises also a certain amount of water bound in this polymer. Due to this reason it is necessary to define also the relative humidity of the air as the drying medium. The drying can be run at the temperatures of 20 °C to 80 °C, and at the relative humidity of air of 15 % to 80 %, preferably by hot air.

In a preferred embodiment the dried filtration cake is then inserted into a metallization bath, then the fibres are collected from the metallization bath, washed, and dried.

Further the invention relates to a wound cover comprising the above mentioned biopolymer staple fibres, the fibres being prepared by the method described above, or the mixture of fibres prepared by the method described above and the fibres selected from the group comprising the fibres based on the natural mycelium processed by a method maintaining the fibre character of natural mycelium, i.e. the material from the natural mycelium has the character of a fibre with the diameter of 1 to 100 µm and the length at least of 0.1 mm. Biopolymer staple microfibers of internal and/or external wounds cover have a diameter of 1 to 100 µm and the length at least of 0.8 cm, preferably the length within the range of 0.8 to 3 cm. The fibres from which the cover is prepared can be non-shortened, i.e. of the length of 0.8 to 3 cm, or shortened in a shortening bath comprising C₁-C₃ alcohol or a mixture of C₁-C₃ alcohol and an organic acid, to the length suitable for the method of preparation of a nonwoven textile, i.e. the length for example of 1 to 5 mm.

The wound cover is preferably in the form of nonwoven textile, nonwoven textile fastened on a pad, cotton wool, tampon, or paper stuff. An apparatus suitable for carrying out the method of the present invention comprises a cylindrical container for the coagulation bath, and a rotary disc with the vertical axis of rotation passing through the bottom of the cylindrical container in a distance from the vertical axis of the cylindrical container. The apparatus further comprises a spinning nozzle the outlet of which is arranged in the cylindrical container in a distance from its wall, the distance being 70 to 130 % of the shortest distance of the rotary disc from the wall of the cylindrical container. The rotary disc diameter is preferably 10 to 30 % of the diameter of the cylindrical container. In a preferred embodiment of the invention the angle spacing of the vertical axis of the rotary disc and the outlet of the spinning nozzle is above 80°, with respect to the vertical axis of the container. In another preferred embodiment of the invention the spinning nozzle can be bent on its end comprising the outlet.

The invention will be described in more detail in the following description.

Biopolymer, as disclosed herein, is preferably a polysaccharide with the size of macromolecules of 60 kDa to 3 MDa (determined by the conventional SEC-MALLS, Size-Exclusion Chromatography coupled to Multi-Angle Laser Light Scattering method, this method is disclosed in detail for example in the publication: Bezáková, Z. et al., Effect of microwave irradiation on the molecular and structural properties of hyaluronan. Carbohydrate Polymers 2008, 73, (4), 640 - 646). Polysaccharides used in the present invention are:
1. Hyaluronic acid and/or at least one compound comprising hyaluronic acid and metal ions.
   The term "a compound comprising hyaluronic acid and metal ions" comprises any compound comprising hyaluronic acid and metal or metals ions, including salts and metal complex compounds. The character of the bond between hyaluronic acid and a metal depends on the metal, usually it is an ionic bond, a covalent bond (ion-covalent) or a coordinate covalent bond. The said term also comprises the compounds comprising ions of more than one metal. The metals are preferably selected from the group comprising lithium, sodium, potassium, calcium, magnesium, copper, manganese, zinc, chromium, iron, and cobalt.
2. Schizophylan
3. Chitin/chitosan-glucane complex and/or at least one compound comprising chitin/chitosan-glucane complex and metal ions. The term "a compound comprising chitin/chitosan-glucane complex and metal ions" comprises any compound comprising chitin/chitosan-glucane complex and metal or metals ions, including salts and metal complex compounds. The character of the bond between chitin/chitosan-glucane complex and metal depends on the metal, usually it is an ionic bond, a covalent bond (ion-covalent) or a coordinate covalent bond. The said term also comprises the compounds comprising ions of more than one metal. The metals are preferably selected from the group comprising lithium, sodium, potassium, calcium, magnesium, copper, manganese, zinc, chromium, iron, and cobalt.
4. Internal mixture of chitin and chitosan and/or at least one compound comprising the internal mixture of chitin and chitosan and metal ions. The term "a compound comprising the internal mixture of chitin and chitosan and metal ions" comprises any compound comprising the internal mixture of chitin and chitosan and metal or metals ions, including salts and metal complex compounds. The character of the bond between the internal mixture of chitin and chitosan depends on the metal, usually it is an ionic bond, a covalent bond (ion-covalent) or a coordinate covalent bond. The said term also comprises the compounds comprising ions of more than one metal. The metals are preferably selected from the group comprising lithium, sodium, potassium, calcium, magnesium, copper, manganese, zinc, chromium, iron, and cobalt.
5. Sodium, potassium, or ammonium alginate.
6. Xanthan and/or its sodium or potassium salt.
7. Oxycellulose and/or its sodium or potassium salt.

The term "pure C₁-C₃ alcohol" refers to C₁-C₃ alcohol of the concentration of 97.5 to 100 %, which is the starting concentration at the beginning of the spinning.

The method of preparation of staple microfibres according to the present invention is based on the use of a non-stationary coagulation bath, and its principle consists in the preparation of a spinning solution (preferably water solution) comprising a biopolymer or a mixture of biopolymers, and said solution is then spun in a non-stationary coagulation (precipitation) bath consisting of 97.5 to 100% C₁-C₃ alcohol, preferably propane-2-ol. During the spinning the concentration of alcohol decreases due to adding of the spinning solution. Optimum conditions are achieved if the water content varies from 0 to 40 % by weight, preferably 10 % by weight.

The coagulation bath does not comprise an acid. We have found by our own study that when the coagulation bath does not comprise an acid, the fiber formation at the nozzle outlet slows down, so the microfibers of the length up to 3 cm are formed and, in addition, the fibres do not stick together.

The term "coagulation bath" refers to a bath where the microfibers are formed from a spinning solution. This bath can be also denoted as the "spinning bath" or "precipitation bath".

In the case of water soluble biopolymers the following method is used: first a water solution of biopolymer with pH in the range of 1 to 13, or any mixture of biopolymers, where the concentration of biopolymer (or the sum of biopolymers) in this solution is 0.01 to 8 % by weight, is prepared, and this solution is then spun by the nozzle into a non-stationary coagulation bath.

The water solubility of the biopolymers without -COOH group can be enhanced by adding urea in such a way that its concentration in the spinning solution is 2 to 15 % by weight.

In another embodiment the spinning solution can be agitated for 10 minutes to 1 hour, untill the formation of a homogenous solution suitable for spinning. Thereafter, this solution can be preferably stored for 5 to 24 hours in a refrigerator at the temperature of -40°C to -10 °C. Then the solution is taken out, let heat to the room temperature, and this solution is then spun through the nozzle into a non-stationary coagulation bath. The low temperature causes disruption of the net of inner and outer hydrogen bonds of biopolymer, so the individual molecules separate apart and "linearize", so they spin better from the solution (this technique is described for example for cellulose in the articles: Ying Wang, Yulin Deng. The Kinetics of Cellulose Dissolution in Sodium Hydroxide Solution at Low Temperatures. Biotechnology and Bioengineering, 102, 5, 2009; Magali Egal, Tatiana Budtova, Patrick Navard. The dissolution of microcrystalline cellulose in sodium hydroxide-urea aqueous solutions. Cellulose, 15, 361, 2008).

A special issue is the preparation of a spinning solution of chitin-chitosan complex with its content of 0.01 to 8 % by weight, where chitin is dissolved in the mixture of dichloroacetic acid and formic acid or acetic acid (1:1 by weight), chitosan is dissolved in formic acid or acetic acid, and both the solutions are combined at agitation. The formed homogenous solution is then spun through the nozzle into a non-stationary coagulation bath.

In this spinning method the forming microfibre is formed and drawn by the force of a stream of the coagulation bath. Fibre drawing is a process where the fibre is deformed (elongated) along the longitudinal axis of the fibre. The length of the fibre increases, its diameter decreases, and its sheerness increases untill forming a microfibre. Due to the orientation of macromolecules and supramolecular forms, and due to the increasing of the crystalline fraction, the mechanical parameters of the fibre enhance: its strength increases and its elongation decreases. During the process of elongation, less stable crystalline modifications can also change into more stable modifications.

The spinning can be carried out also by the dry-wet method, where an air passage before the entry of the stream of the solution being spun into the coagulation (spinning) bath is 1 to 200 mm.

The inner diameter of the nozzle is, of course, larger than in the order of µm because it would not be possible to press the solutions, which are relatively viscous, through the nozzle (needle) so narrow.

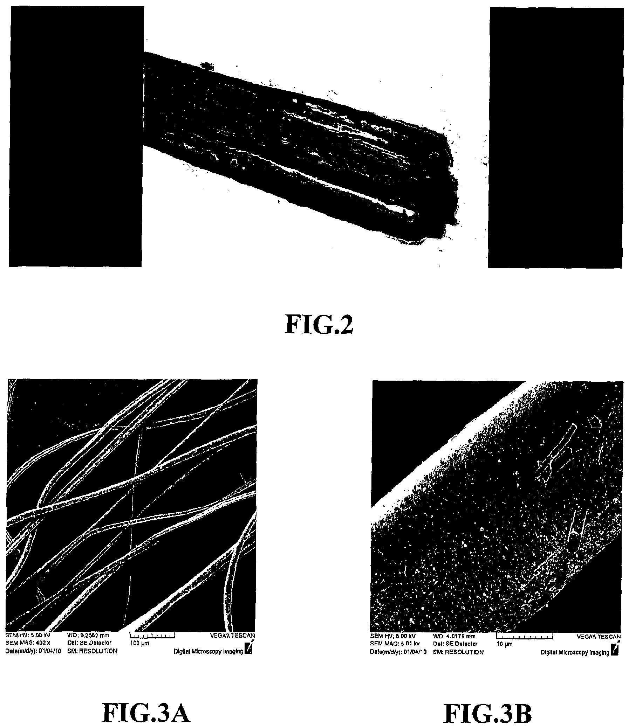

We have found by our own study that in the stream leaving the nozzle, a microfibre bundle is formed in the coagulation bath, these microfibers separate apart in the moving coagulation bath (of course, provided that the twisting thereof does not occur). Fig. 2 shows, by way of example, a bundle of primary microfibers of schizophyllan, displayed under the optical microscope. Other fibre-forming polysaccharides behave in a similar way.

Afterwards, the microfibres are removed (filtrated) from the coagulation bath and their strength is enhanced in a maturation bath. The maturation bath can comprise, in addition to the alcohol, also an acid, which causes, for example in the case of hyaluronan, a change of COONa group into -COOH group and thus forms a system of hydrogen bonds and therefore causes the formation of a tight supramolecular structure. Since the fibrils could be not tight enough, which can happen due to the absence of an acid in the coagulation bath, the formed microfibers are immersed to the maturation bath for 20 minutes to 96 hours. The maturation bath preferably has the composition selected from the group comprising a mixture consisting of 90 to 100 % by weight of a C₁-C₃ alcohol, and up to 10 % by weight of an organic acid, for example formic acid or acetic acid. The bath therefore comprises either an alcohol alone or it can also contain an acid, which helps to stabilize the fibre structure, and therefore the fibre is tighter.

It is advisable to collect the forming microfibre form the bath immediately to prevent its uncontrollable twisting. The collected fibre can be inserted to a maturation bath. In the maturation bath, the system of hydrogen bonds, having been broken by dissolving of a biopolymer, is reconstituted, or, in other words, a secondary and tertiary structure (supramolecular in general) of a biopolymer, bearing the necessary biological and mechanical characteristics, is restored. It can be concluded that in the coagulation bath the microfibre of suitable dimensions is prepared, and then in the maturation bath the right mechanical characteristics are "prepared".

As the microfibers formed in the non-stationary coagulation bath are too long for many applications, they can be preferably "shortened" to the length of approximately 1 to 5 mm in an appropriate knife mixer with the use of the maturation bath as the medium. A new bath, the so-called shortening bath, can also be used. The composition of the shortening bath is the same as the coagulation bath or the maturation bath. The shortening is necessary for the following preparation of planar textiles, not for the preparation of cotton wool.

Then it is optionally possible to further homogenize the fibres formed in this way with the aid of a suitable high speed dispergator in the so-called homogenization bath, where the previous shortening bath is preferably used. A new bath, the so-called homogenization bath, can be also used. The composition of the homogenization bath is the same as the coagulation bath and/or maturation bath.

The prepared staple microfibers are then collected/filtrated either alone in a suitable filtration apparatus or they are filtrated (fastened) onto a pad, generally a textile, optionally washed with alcohol and dried, again alone or together with the pad. The microfibers layer can be optionally removed from the pad. If it is presumed to remove the pad, it is advisable to use a more hydrophobic pad, for example low molecular polyethylene, which does not form H-bonds with the given fibres.

In another embodiment the microfibres are filtrated onto a pad, optionally washed, and fastened to this pad by wet compression with the use of a suitable apparatus, and dried (together with the pad).

The drying temperature of prepared staple microfibers is 20 to 80 °C.

In another embodiment the microfibers of various chemical composition obtained by the previous procedures are mixed together in any ratio in the shortening bath or preferably in the homogenization bath, and they are homogenized in the homogenization bath. The following procedure is then the same as for the previous processes.

In this invention, the alcohol is methanol, ethanol, propane-l-ol, propane-2-ol, or any mixture thereof. The acid is formic acid, acetic acid, propionic acid, or any mixture thereof.

In another embodiment the microfibers obtained by the previous processes can be placed into the so-called metallization bath for 20 minutes to 25 days, where various metals are inserted into the microfibers; in this invention it concerns metal ions of calcium, magnesium, copper, manganese, zinc, chromium, iron, and cobalt. From the technological point of view the metallization can be carried out in every bath (coagulation, maturation, shortening, homogenization; in the case of zinc also in the spinning solution) by adding a salt of polyvalent metal into a proper bath, or a separate metallization bath can be prepared, where this technological step (metallization) can be added to any step, right from the metallization immediately after the coagulation. The metallization is most commonly carried out in the maturation bath. If the metallization step is present, it is necessary to wash the final fibres with alcohol, for example with methanol or isopropanol.

In the case of biopolymers having the sequestration properties (hyaluronic acid/hyaluronan, internal mixture of chitin and chitosan, chitin/chitosan-glucane complex), the invention is characterized in that the metallization bath is
a) a solution of the salt of polyvalent metal of the concentration of 5.10⁻⁵ to 1.10⁻² M in the mixture of 1 to 99 % by weight of acid, 1 to 99 % by weight of alcohol and up to 10 % by weight of water, or
b) a solution of the salt of polyvalent metal of the concentration of 5.10⁻⁵ to 1.10⁻² M in the alcohol comprising up to 10 % by weight of water, optionally in the presence of a base selected from the group comprising LiOH, NaOH, KOH, Li₂CO₃, Na₂CO₃, K₂CO₃, LiHCO₃, NaHCO₃ a KHCO₃, with the concentration of the base of 5.10⁻⁵ to 1 N.

As the salts of the polyvalent metal (Mn²⁺, Ca²⁺, Cr³⁺, Fe²⁺, Fe³⁺, Co²⁺, Co³⁺, Cu²⁺, Zn²⁺), their acetates, formates, chlorides, sulphates, fluorides, nitrates, and hydrates are meant.

The prepared microfibres are usable as a free cotton wool, further for the production of nonwoven textiles of various types and by various techniques, and, after carding, they can be also used for the production of staple yarn. Nonwoven textiles, cotton wool, tampons, paper stuff, etc. can be made from the fibres.

The produced nonwoven textile can be "self-supporting" or in the form of a layer of microfibers on a suitable carrier/pad (e.g. textile, preferably fabric or knitted fabric) for the use for suitable wound covers.

The fastening of microfibres on a suitable textile is a substitution of lyophilization, which is technologically very complicated and expensive.

The method of spinning according to the present invention in the non-stationary coagulation bath and the following treatments in the consecutive baths exhibits certain advantages. The first advantage is that due to the staple microfibre, which is long enough, it is possible to prepare a nonwoven textile with a very low areal weight (∼ 5 g/m²) and at the same time tight enough for the common manipulation with the nonwoven textile of this areal weight. The nonwoven textile of the areal weight from 10 g/m² can be advantageously used as self-supporting, i.e. without any pad, for example as a cover of internal wounds.

A further benefit is that during the spinning, at a low concentration of a polymer, e.g. 0.5 to 1 % by weight of the aqueous polymer solution, it is possible to prepare the spinning solution only in water and spin it e.g. into propane-2-ol. Due to the fact that NaOH is not added into the spinning solution, the medium is not alkaline, thus the polymer does not degrade. In addition, the complicated removal of organic acids (formic, acetic) and alcohol (methanol, ethanol) from the fibre prepared by the conventional method (PV 2010-1001) is avoided.

During the spinning of e.g. internal mixture of chitin and chitosan we avoid using the coagulation bath comprising substantial amounts of inorganic salts (10 % of NaOH and 30 % of sodium acetate). Furthermore, a substantial advantage of this method is that during the spinning in the coagulation bath comprising generally an alcohol (for example propane-2-ol), the ammonium groups (chitosan-NH₃⁺X⁻, X⁻=Cl⁻, CH₃COO⁻, etc.) are not converted to amine groups and therefore the microfibres keep their haemostatic and antibacterial effect.

A certain amount of solvent used during the production process remains in the final microfibers/fibers. This amount can be defined with the aid of NMR spectroscopy within the range of 0.2 to 5 % by weight.

The subject of the invention further comprises covers of internal and external wounds based on the microfibers prepared by the method of the invention. The covers of internal and external wounds can be in the form of woven or nonwoven textiles or cotton wool. The covers of internal and external wounds are for example dressings, bandages, patches, tampons.

The subject of the invention therefore comprises the covers of internal and external wounds based on staple microfibres consisting of hyaluronic acid and/or at least one compound comprising hyaluronic acid and metal ions, where the fibre can optionally comprise metal salts originating from the production process, which can be prepared by the method according to the present invention, which has the properties of a textile fibre, i.e. nonwoven textiles, cotton wool, tampons, paper stuff, and the like can be made from this fibre. The fibre is composed of hyaluronic acid and/or at least one compound comprising hyaluronic acid and metal ions, where the molecular weight of macromolecules is within the range of 100 kDa to 3 MDa. Preferably the diameter of microfibre (primary fibre) is 1 - 100 µm and the length 0.8 - 3 cm.

The term "a compound comprising hyaluronic acid and metal ions" includes any compound comprising hyaluronic acid and metal or metals ions including salts and metal complex compound. The character of the bond between hyaluronic acid depends on the metal, usually it is an ionic bond, covalent (ionic covalent) or coordinate covalent bond. The said term also comprises the compounds comprising the ions of more than one metal. The metals are preferably selected from the group comprising lithium, sodium, potassium, calcium, magnesium, copper, manganese, zinc, chromium, iron, and cobalt.

Preferably, the maximum content of these cations in a fibre is as follows:

| Metal ion | Maximum content in a fibre (% hm.) |
|---|---|
| Li+ | 1.80 |
| Na+ | 5.72 |
| K+ | 9.36 |
| | |
| Mg2+ | 3.11 |
| Ca2+ | 5.03 |
| | |
| Mn2+ | 6.77 |
| Zn2+ | 7.95 |
| Fe2+ | 6.87 |
| Cu2+ | 7.75 |
| Co2+ | 7.22 |
| | |
| Cr3+ | 4.38 |
| Fe3+ | 4.69 |
| Co3+ | 4.93 |

The fibre can optionally comprise a metal salt originating from the production process and it refers to acetates, formates, sulphates, chlorides, fluorides, nitrates, phosphates, hydroxides, or oxides of the metals selected from the group comprising lithium, sodium, potassium, calcium, magnesium, copper, manganese, zinc, chromium, iron, and cobalt. These salts can form a weak or strong interaction with hyaluronic acid. In the case of zinc, the fibre can also comprise the zinc salts Zn²⁺ as the metal salts. The wound covers based on staple microfibers of hyaluronic acid/hyaluronan serve for the construction of bandages absorbing the liquid from the wound and at the same time they stimulate the process of granulation. In the combination with a suitable antiseptic agent they can be utilized for both the acute and chronic non-healing wounds.

Further, the subject of the invention comprises the covers of internal and external wounds based on staple microfibres consisting of chitin/chitosan-glucan complex, wherein the fibre can optionally comprise metal salts originating from the production process, which can be prepared by the procedure according to the present invention, and has the properties of a textile fibre, i.e. both the woven and nonwoven textiles, cotton wool, tampons, paper stuff, and the like can me made from this fibre. The microfibre/fibre is composed of chitin/chitosan-glucane complex, where the molecular weight of macromolecules is within the range of 70 kDa to 1 MDa. Wound covers based on the staple microfibers of chitin/chitosan-glucane complex serve to cover acute seriously contused and bleeding wounds before their final treatment. They can be of use also in the case of wounds which tend to form necrotic crusts.

Further, the subject of the invention comprises the covers of internal and external wounds based on staple microfibres consisting of schizophylane, wherein the fibre can optionally comprise metal salts originating from the production process, which can be prepared by the procedure according to the present invention, and has the properties of a textile fibre, i.e. both the woven and nonwoven textiles, cotton wool, tampons, paper stuff, and the like can be made from this fibre. The microfibre/fibre is composed of schizophylane, where the molecular weight of macromolecules is within the range of 200 kDa to 2 MDa. These wound covers can be utilized in combined bandages (wound covers) at the patients who need improvement of local immune processes (e.g. the patients treated with corticosteroids or other immuno suppressants).

We have found by our own study that amino groups play the key role in spinning of chitin or chitosan. In our opinion, the type of supramolecular structure (helix, double or triple helix, fibres, gels, etc.) depends on the presence of amino groups in the macromolecule. The amino group, due to its sp³ hybridization, forms 3D structures via the hydrogen bonds. Therefore, it has 3D cross-linking character and thus, these molecules preferentially form gels in water, which are, however, not easy to spin properly. On the contrary, the acylated amino groups, due their H-bonds, form 2D structures, so they support the formation of linear chains (for example double-helixes) suitable for spinning. As it was stated above, the chitin amino groups are mainly acetylated whereas the chitosan amino groups are deacetylated (in other words, there are predominantly the amino groups). The problem consists in that chitin can be spun, but it has not the required biological properties. On the contrary, chitosan has proper biological properties, but it is not possible to prepare fibres from it, or they are of low quality (fragile). The solution is provided by the present invention, where chitin with suitable spinning properties is used on purpose. Further the chitosan with suitable biological properties is used. The principle consists in dissolving both the polymers, mixing the solutions (in other words, two types of macromolecules are mixed to form chitin-chitosan complex) so that the weight ratio of chitin/chitosan is within the range of 3/4 - 7/1, and the obtained homogenous solution is spun in the coagulation bath.

The subject of the invention further comprises covers of internal and external wounds from staple fibres consisting of an internal mixture of chitin and chitosan (i.e. chitin-chitosan complex) that can be prepared by the method according to the present invention, the fibres having the properties of a textile fibre, which means that nonwoven textiles, cotton wool, tampons, paper stuff, and the like can be made from this staple microfibre. The staple microfibre is composed of chitin, where the molecular weight of the macromolecules is within the range of 100 kDa to 1 MDa, and chitosan, where the molecular weight of the macromolecules is within the range of 60 kDa to 1 MDa. These wound covers will be used for the construction of haemostatic and antibacterial bandages.

### Brief description of the drawings

Fig. 1A is a schematic section plan of the apparatus for the preparation of staple microfibres.
Fig. 1B is a vertical section of the apparatus from the Fig. 1A.
Fig. 1C shows a straight nozzle (needle).
Fig. 1D shows a bent nozzle (needle).
Fig. 2 shows a bundle of schizophylane microfibres.
Fig. 3 shows the character of the fibres prepared according to the Example 1 (Fig. 3A - magnification of 400x; Fig. 3B - magnification of 5000x).
Fig. 4 shows a nonwoven textile prepared in the Example 2.
Fig. 5 shows a cotton wool prepared in the Example 3.
Fig. 6 shows the character of the fibres prepared according to the Example 4 (magnification of 500x).
Fig. 7 shows the character of the fibre layer prepared according to the Example 5 (magnification of 500x).
Fig. 8 shows a microphotography of the layer prepared according to the Example 12 (magnification of 1000x).
Fig. 9 shows a microphotography of the layer prepared according to the Example 13 (magnification of 1000x).
Fig. 10a shows the wound cover prepared according to the Example 14 - self-supporting, possible to be applied without any supporting knitted fabric.
Fig. 10b shows the character of a fibre structure (wound cover) prepared from the staple microfibres of hyaluronic acid and native microfibres of chitin/chitosan-glucan complex in the ratio of 1:1 according to the Example 14.
Fig. 11 shows the wound cover prepared according to the Example 16 (on a supporting textile).
Fig. 12a shows the wound cover prepared according to the Example 16 (self-supporting layer).
Fig. 12b shows the wound cover according to the Example 16. The layer character under the electron microscope. Magnification of 1000x.
Fig. 13 shows the wound cover prepared according to the Example 22. The layer character under the electron microscope. Magnification of 1000x.

### Preferred embodiments of the invention

The pictures of the fibres were made with the microscope Tescan VEGA II LSU (Tescan, Brno).

This microscope utilizes wolfram cathode and the maximum resolution is 3 nm. The parameters of measurement were as follows: accelerating voltage of the primary electron beam: 5 kV, working distance - WD: 4 - 5 mm, pressure in the chamber: high vacuum, display mode: secondary electrons.

The fibres were stuck on a carbon sticking target and then powdered with gold. The gold layer on the sample: approx. 15 nm, powdering machine: SC7620 Mini Sputter Coater (Quorum Technologies, UK).

The molecular weight of hyaluronic acid, or hyaluronans, was measured with the aid of HPLC from Shimadzu, equipped with the light scattering detector miniDAWN from Watt Technologies (the so-called SEC-MALLS method).

The apparent viscosity was measured with ARG2 rheometer from TA Instruments. The cone (40 mm/1°)-plate system of measurement was used. Cone data: first - diameter, second - bevel angle.

The metals were determined with the aid of sequential optical emission spectrometer with the ionization in the inductively coupled plasma (Integra XL2, GBC Australia) with the use of ceramic V-groove nebulizer and cyclonic cloud chamber (both from Glass Expansion, Australia).

### Example 1

In the air atmosphere, at intense stirring, 6 grammes of sodium hyaluronan with molecular weight of 1.7 MDa (determined by the SEC-MALLS method) was dissolved in 94 g of water with adding of 0.51 g NaOH to obtain a homogenous, well flowing, viscous solution suitable for spinning. This solution was spun by the wet method with a nozzle having the diameter of 0.4 mm into the spinning bath comprising propane-2-ol, stirred with an agitator, more specifically with the Heidolph DIAX 900 dispergator at 2500 rpm. The forming microfibre was collected from the "moving (non-stationary) bath" and transferred to the "maturation bath" consisting of propane-2-ol, where the coagulation of the fibre was completed. After 60 minutes, the microfibre was removed from the maturation bath and dried at the temperature of 60 °C. The fibre character is shown in Fig. 3. The length of the microfibres obtained by this method is approximately 5 - 30 mm. This type of fibres is suitable for the construction of bandages which absorb the liquid from the wound and at the same time they stimulate the process of granulation. In the combination with a suitable antiseptic agent they can be utilized for both the acute and the chronic non-healing wounds. Separately, they can be used for the application into caverns or defects after the teeth extraction. They can also be useful in the field of plastic and correction surgery.

### Example 2

In the air atmosphere, at intense stirring, 6 grammes of sodium hyaluronan with the molecular weight of 1.7 MDa (determined by the SEC-MALLS method) was dissolved in 94 g of water with adding of 0.51 g NaOH to obtain a homogenous, well flowing, viscous solution suitable for spinning. This solution was spun by the wet method with a nozzle having the diameter of 0.4 mm into the spinning bath comprising propane-2-ol, stirred with the Heidolph DIAX 900 dispergator at 2500 rpm. The forming microfibre was collected from the "moving bath" and transferred to the "maturation bath" consisting of propane-2-ol, where the coagulation of the fibre was completed.

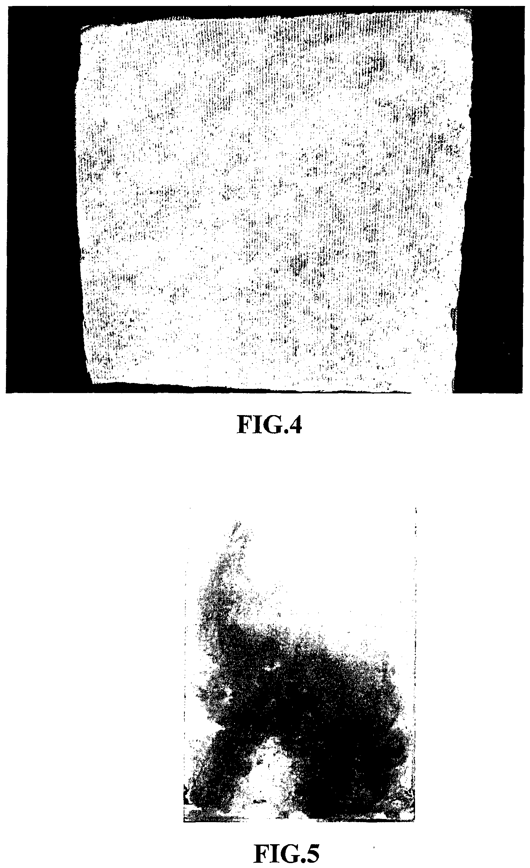

After 60 minutes the maturation bath containing the microfibres was poured into the Eta-Ergo knife mixer, where the fibres were shortened. Then the shortened fibre was filtrated off and the obtained filtration cake was compressed to the form of a "paper" sheet, and the produced product was dried at the room temperature (approx. 25 °C). The paper sheet, or nonwoven textile, is shown in Fig. 4.

The length of the microfibres obtained by this method is approximately 5 - 30 mm.

This textile is suitable for flat absorption bandages which stimulate the granulation and wound healing. In the combination with an antiseptic agent they can be used for the construction of functional bandages and patches. They can also be useful in the field of plastic and correction surgery.

### Example 3

In the air atmosphere, at intense stirring, 6 grammes of sodium hyaluronan with the molecular weight of 1.7 MDa (determined by the SEC-MALLS method) was dissolved in 94 g of water with adding of 0.51 g NaOH to obtain a homogenous, well flowing, viscous solution suitable for spinning. This solution was spun by the wet method with a nozzle having the diameter of 0.4 mm into the spinning bath comprising propane-2-ol, stirred with the Heidolph DIAX 900 dispergator at 2500 rpm. The forming microfibre was collected from the "moving bath" and transferred to the "maturation bath" consisting of propane-2-ol, where the coagulation of the fibre was completed.

After 60 minutes the maturation bath containing the microfibres was poured into the Eta-Ergo knife mixer, where the fibres were shortened. Then the shortened fibre was filtrated off and the filtration cake was dried without compression at 20 °C. A voluminous layer of microfibres - a cotton wool showed in Fig. 5 - was thereby obtained as a base of a nonwoven textile suitable for various uses.

The cotton wool is suitable for the direct application into deep wounds and caverns or defects after the teeth extraction. It will also be of use in surgery for improving the healing of various connections and anastomoses. For this purpose, the combination of fibres and an appropriate antiseptic agent is possible.

### Example 4

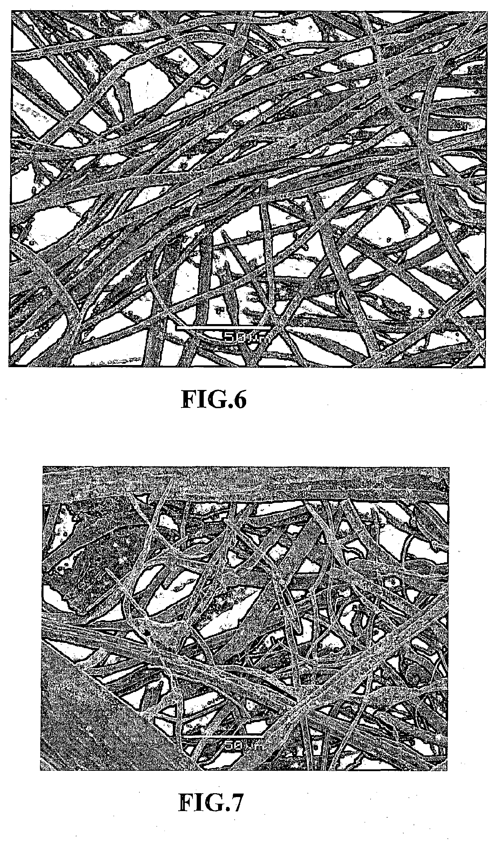

In the air atmosphere, at intense stirring, 6 grammes of sodium hyaluronan with the molecular weight of 19.37 MDa (determined by the SEC-MALLS method) was dissolved in 94 g of water to obtain a homogenous, well flowing, viscous solution suitable for spinning. This solution was spun by the wet method with a nozzle having the diameter of 0.4 mm into the spinning bath comprising propane-2-ol, stirred with the Heidolph DIAX 900 dispergator at 2500 rpm. The forming microfibre was collected from the bath and transferred to methanol where the coagulation of the fibre was completed. After 60 minutes the fibre was dried at the temperature of 60 °C and its character is shown in Fig. 6.

The use of this fibre is the same as in the Example 1.

### Example 5

In the air atmosphere, at intense stirring, 6 grammes of sodium hyaluronan with the molecular weight of 19.37 MDa (determined by the SEC-MALLS method) was dissolved in 94 g of water to obtain a homogenous, well flowing, viscous solution suitable for spinning. This solution was spun by the wet method with a nozzle having the diameter of 0.4 mm into the spinning bath comprising propane-2-ol, stirred with the Heidolph DIAX 900 dispergator at 2500 rpm. The forming microfibre was collected from the bath and transferred to methanol where the coagulation of the fibre was completed. After 60 minutes the maturation bath containing the microfibres was poured into the Eta-Ergo knife mixer, where the fibres were shortened. Then the shortened fibre was filtrated off, compressed on a calender (with medium pressure) and dried at the room temperature (approx. 25 °C). The character of the layer is shown in Fig. 7.

A voluminous layer of microfibres, suitable for the production of nonwoven textile, is thereby obtained.

This textile is suitable for flat absorption bandages which stimulate the granulation and wound healing. In the combination with an antiseptic agent it can be used for the construction of functional bandages and patches. They will be of use especially for healing of extensive defects. It concerns especially the broken surgical wounds and polytraumas.

### Example 6

In the air atmosphere at intense stirring, 3 g of chitosan from Aldrich company (catalog no. 448869 - declared as: Chitosan, low molecular weight, 75-85 % deacetylated) were dissolved in 72 g of 2% acetic acid (water solution) to obtain a 4% homogenous, well flowing viscous solution suitable for spinning. In this solution, 3 g of sodium hyaluronan (Contipro Biotech s.r.o.) with the molecular weight of 19.37 kDa (determined by the SEC-MALLS method) was dissolved. The obtained solution was spun by the wet method with a nozzle having the diameter of 0.6 mm into the spinning bath comprising propane-2-ol and stirred with the Heidolph DIAX 900 dispergator at 2200 rpm.

The forming microfibre was collected from the bath and transferred to methanol where the coagulation of the fibre was completed. After 60 minutes the maturation bath containing the microfibres was poured into the Eta-Ergo knife mixer, where the fibres were shortened. Then the shortened fibre was filtrated off and the obtained filtration cake was dried at 20 °C without compression.

This bandage is suitable for acute bleeding wounds. It will be of use especially in traumatology.

### Example 7

In the air atmosphere at intense stirring, 1 g of xanthane (Xanthan Gum - Donau Chem-Typ: 890.235, 402 80 MESH, Ceroga Pharm) was dissolved in 99 g of water to obtain a 1% homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened. Then the shortened fibre was filtrated off and the obtained filtration cake of xanthane fibres was dried at 20 °C without compression.

The xanthane fibres can adjust the adhesiveness and hydration character of combined bandages.

### Example 8

In the air atmosphere at intense stirring, 0.5 g of xanthane (Xanthan Gum - Donau Chem-Typ: 890.235, 402 80 MESH, Ceroga Pharm) and 0.5 g of hyaluronic acid (Contipro Biotech s.r.o. Dolní Dobrouč; 1.7 MDa, determined by the SEC-MALLS method) were dissolved in 99 g of water to obtain a 1% homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened. The shortened fibre was filtrated off and the obtained filtration cake of mixed fibres (xanthane-hyaluronic acid) was dried at 20 °C without compression.

The xanthane fibres in the combination with hyaluronan can modify the flow of interstitial fluid and thereby regulate the adhesiveness and hydration character of combined bandages.

### Example 9

In the air atmosphere at intense stirring, 3 g of ammonium alginate (Protamin S - fy. FMC BioPolymer, N-3002 Drammen, Norway) was dissolved in 97 g of water to obtain a 3% homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened. The shortened fibre was filtrated off and the obtained filtration cake of the fibre of ammonium alginate was dried at 20 °C without compression.

The alginate staple fibres can be used for example for entrapping the fluid in the area of a dry wound.

### Example 10

In the air atmosphere at intense stirring, 1 g of ammonium alginate and 1 g of sodium hyaluronan (Contipro Biotech s.r.o. Dolni Dobrouč; 1.7 MDa, determined by the SEC-MALLS method) were dissolved in 98 g of water to obtain a 2% homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened. The shortened fibre was filtrated off and the obtained filtration cake of the mixed fibre of alginate - hyaluronic acid was dried at 20 °C without compression.

These mixed fibres ensure various hydration and granulation characteristics of the wounds with impaired vascularity.

### Example 11

In the air atmosphere at intense stirring, 0.25 g of schizophylane (Contipro Biotech s.r.o. Dolní Dobrouč; 1.7 MDa, determined by the SEC-MALLS method) was being dissolved in 25 g of water for 24 hours to obtain a homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm.

The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and further they were shortened and homogenized with the aid of Heidolph DIAX 900 mixer. A layer of this fibre was prepared by the filtration on a suitable pad (knitted fabric from polyamide 6 (silk) with areal weight of approx. 40 g.m⁻²). The obtained filtration cake was compressed (together with the pad) in a two-roll calender and dried at 20 °C. As the layer obtained in this way has no affinity to the pad, it gets unstuck spontaneously during the drying. The areal weight of the obtained fibres (nonwoven textile) from schizophylane was approx. 15 g/m². The formed microfibre layer can be applied including the pad or it can be removed - separated - from the pad and the layer of microfibres can be used separately.

The fibres obtained thereby are of use in combined bandages for the patients who are in need of improvement of local immune processes (e.g. the patients treated with corticosteroids or other immunosuppresants).

### Example 12

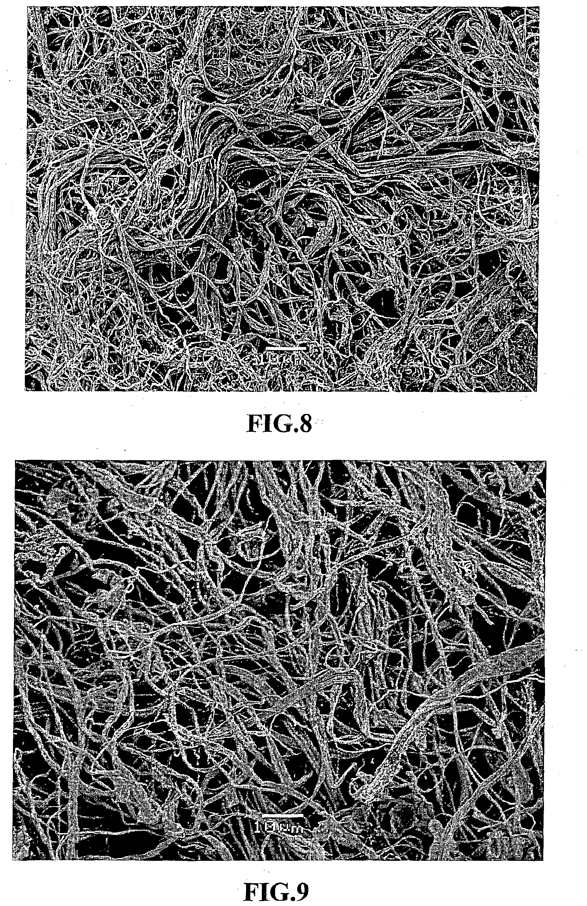

In the air atmosphere at intense stirring, 0.25 g of schizophylane (Contipro Biotech s.r.o. Dolní Dobrouč) and 0.25 g of sodium hyaluronan (Contipro Biotech s.r.o. Dolní Dobrouč) with the molecular weight of 1.7 MDa (determined by the SEC-MALLS method) were being dissolved in 50 g of water for 24 hours to obtain a homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and further they were shortened and homogenized with the aid of Heidolph DIAX 900 mixer. The suspension of the mixed fibre was filtrated on a suitable pad (knitted fabric from polyamide 6 (silk) with areal weight of approx. 40 g.m⁻²) and thereby a layer of this mixed fibre was prepared. The obtained filtration cake was wet compressed (including the pad) in a two-roll calender and dried at 20 °C. As the obtained mixed fibres have an affinity to the pad, the layer was not removed from the pad, and the layer of the mixed fibres from schizophylane and hyaluronic acid had the areal weight of approx. 10 g/m² (weight of the pad was subtracted). The formed layer of microfibres (Fig. 8) can be applied including the pad or it can be removed - separated - from the pad and the layer of microfibres can be used separately.

The combination of schizophylane + HA in mixed fibres is of use for the construction of combined immunomodulating bandages.

### Example 13

In the air atmosphere at intense stirring, 0.5 g of sodium hyaluronan (Contipro Biotech s.r.o. Dolní Dobrouč) with the molecular weight of 1.7 MDa (determined by the SEC-MALLS method) was dissolved in 50 g of water to obtain homogenous, well flowing viscous solution suitable for spinning. This solution was spun by the wet method with a nozzle with the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and further they were shortened and homogenized with the aid of Heidolph DIAX 900 mixer. 0.5 g of homogenized fibrous sample of chitin/chitosan-glucane complex (Contipro Biotech s.r.o. Dolní Dobrouč, species: Aspergillus niger. dry mass: 93.64 %, ash: 0.82 %, GLA content: 13.26 %) was added to the shortened fibre. The suspension of the mixture of fibres was further homogenized and in the end filtrated on a suitable pad (knitted fabric from polyamide 6 (silk) with areal weight of approx. 40 g.m⁻²) and thereby a layer of this mixture of fibres was prepared. The obtained filtration cake was wet compressed in a two-roll calender and dried at 20 °C. The obtained layer of the mixture of fibres from chitosan-glucane complex and hyaluronic acid had the areal weight of approx. 15 g/m² (the weight of the pad was subtracted). The formed layer of microfibres (Fig. 9) can be applied including the pad or it can be removed - separated - from the pad and the layer of microfibres can be used separately.

These fibres are suitable for covering acute seriously contused and bleeding wounds before their final treatment. They can also be of use in the case of wounds which tend to form necrotic crusts.

### Example 14

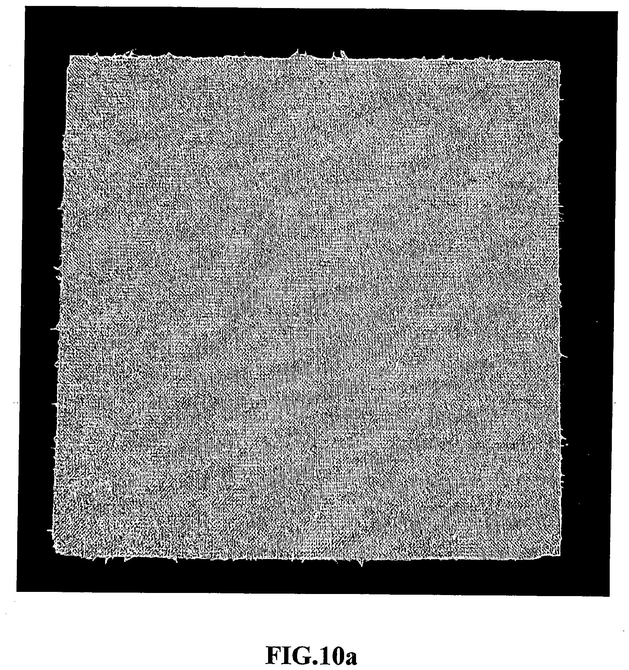

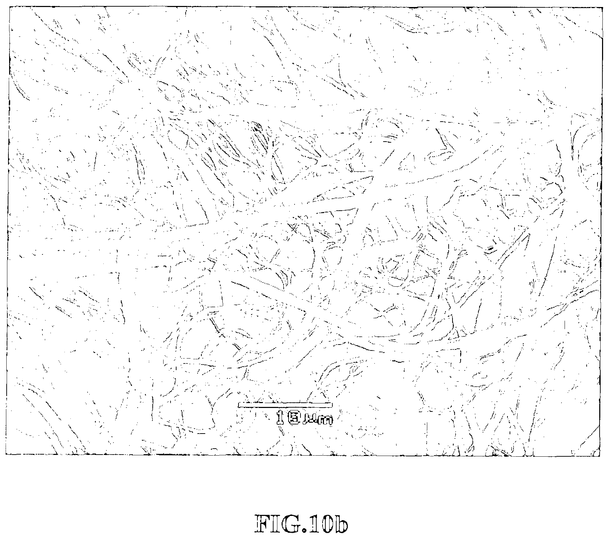

In the air atmosphere at intense stirring, 0.5 g of sodium hyaluronan (Contipro Biotech s.r.o. Dolní Dobrouč) with the molecular weight of 1.7 MDa (determined by the SEC-MALLS method) was dissolved in 50 g of water to obtain a homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and further they were shortened and homogenized with the aid of Heidolph DIAX 900 mixer. 0.5 g of homogenized fibrous sample of chitin/chitosan-glucane complex (Contipro Biotech s.r.o. Dolní Dobrouč, obtained from Schizophyllan commune) was added to the shortened fibre. The suspension of the mixture of fibres was further homogenized and then filtrated on a suitable pad (knitted fabric from polyamide 6 (silk) with areal weight of approx. 40 g.m⁻²) and thereby a layer of this mixture of fibres was prepared. The obtained filtration cake was wet compressed in the two-roll calender and dried at 20 °C and relative air humidity of 15 %. The obtained layer of the mixture of fibres from chitosan-glucane complex and hyaluronic acid had areal weight of approx. 15 g/m² (weight of the pad was subtracted). The formed layer of microfibres can be applied including the pad or it can be removed - separated - from the pad and the layer of microfibres can be used separately (Fig. 10a, 10b).

These fibres are suitable for covering acute seriously contused and bleeding wounds before their final treatment. They can also be of use in the case of wounds which tend to form necrotic crusts.

### Example 15

In the air atmosphere at intense stirring, 0.5 g of sodium hyaluronan (Contipro Biotech s.r.o. Dolní Dobrouč) with the molecular weight of 1.7 MDa (determined by the SEC-MALLS method) was dissolved in 50 g of water to obtain a homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and further they were shortened and homogenized with the aid of Heidolph DIAX 900 mixer. 0.5 g of microfibres from chitin/chitosan-glucane complex (the preparation is described in Example 18) was added to the shortened fibre. The suspension of the mixture of fibres was further homogenized and in the end filtrated on a suitable pad (knitted fabric from polyamide 6 (silk) with areal weight of approx. 40 g.m⁻²) and thereby a layer of this mixture of fibres was prepared. The obtained filtration cake was wet compressed in the two-roll calender and dried at 20 °C. The obtained layer of the mixture of fibres from chitosan and hyaluronic acid had areal weight of approx. 15 g/m² (weight of the pad was subtracted). The formed layer of microfibres can be applied including the pad or it can be removed - separated - from the pad and the layer of microfibres can be used separately.

These fibres are suitable for covering acute seriously contused and bleeding wounds before their final treatment. They can also be of use in the case of wounds which tend to form necrotic crusts.

### Example 16

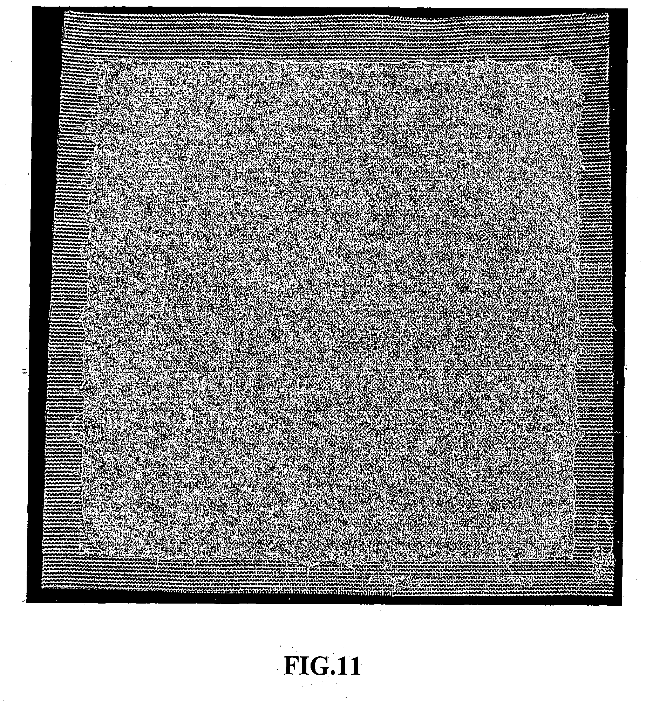

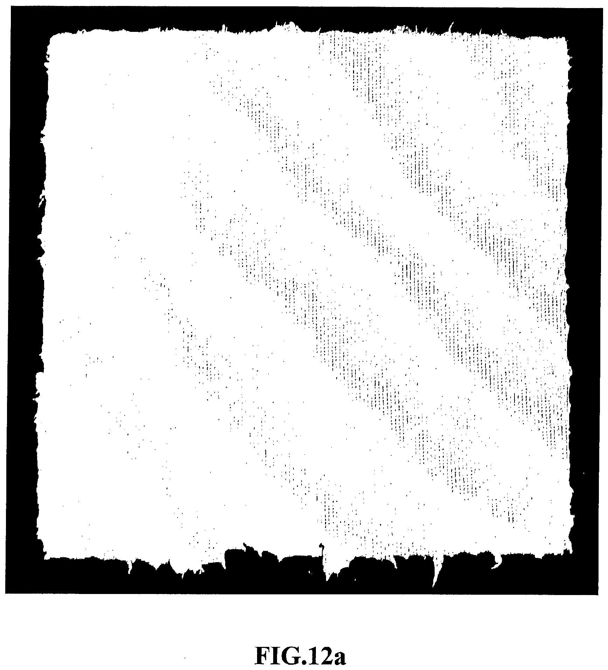

In the air atmosphere at intense stirring, 0.5 g of sodium hyaluronan (Contipro Biotech s.r.o. Dolní Dobrouč) with the molecular weight of 1.7 MDa (determined by the SEC-MALLS method) was dissolved in 50 g of water to obtain a homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and further they were shortened and homogenized with the aid of Heidolph DIAX 900 mixer. The suspension of the fibres was further homogenized and in the end filtrated on a suitable pad (knitted fabric from polyamide 6 (silk) with areal weight of approx. 40 g.m⁻²) and thereby a layer of this mixture of fibres was prepared. The obtained filtration cake was wet compressed in a two-roll calender and dried at 52 °C and relative air humidity of 18 %. The obtained layer of fibres from hyaluronic acid had areal weight of approx. 15 g/m² (the weight of the pad was subtracted). The formed layer of microfibres can be applied including the pad (Fig. 11) or it can be removed - separated - from the pad and the layer of microfibres can be used separately (Fig. 12a).

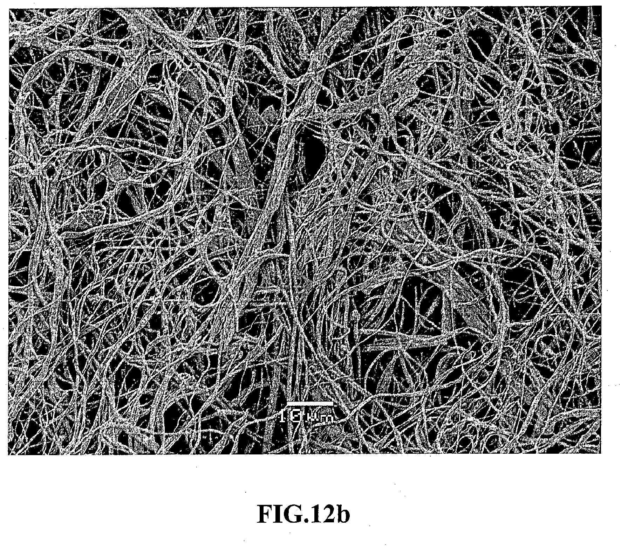

The character of the layer of microfibres under the electron microscope is shown in Fig. 12b.

These fibres are suitable for flat absorption bandages which stimulate the granulation and wound healing. In the combination with an antiseptic agent they can be used for the construction of functional bandages and patches. They can be useful also in the field of plastic and correction surgery.

### Example 17

In the air atmosphere at intense stirring, 1 g of chitosan from Aldrich company (catalog no. 448869 - declared as: Chitosan, low molecular weight, 75-85 % deacetylated) was dissolved in 100 g of 2% acetic acid (2 g of ice-cold acetic acid and 98 g of water) to obtain a 1% homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and further they were shortened and homogenized with the aid of Heidolph DIAX 900 mixer. To this suspension of fibres, the suspension of fibres from hyaluronic acid prepared according to the Example 22 was further added. The suspension of both types of fibres was further homogenized and in the end filtrated on a suitable pad (knitted fabric from polyamide 6 (silk) with areal weight of approx. 40 g.m⁻²) and thereby a layer of this mixture of fibres was prepared. The obtained filtration cake was wet compressed in a two-roll calender and dried at 20 °C.

The obtained layer of fibres from hyaluronic acid and chitosan fibres had areal weight of 15 g/m².

The formed layer of microfibres can be applied including the pad or it can be removed - separated - from the pad and the layer of microfibres can be used separately. The formed layer of microfibres is water-insoluble.

This textile is suitable for the construction of patches for treating smaller wounds and bruises.

### Example 18

In a mixture of acetic acid and formic acid (97 g, mixture 1:1 by weight), 3 g of chitin (University of Pardubice, approx. 1 MDa - determined by viscosimetry), acetylation degree of amine groups approx. 95 %, extracted from crab shells provided by VCI Brasil company) were dissolved at stirring, and the solution was stirred for 2 hours at 22 °C (room temperature).

In another reaction container, 4 g of chitosan (University of Pardubice, approx. 100 kDa - determined by viscosimetry), acetylation degree of amine groups approx. 84 %) were dissolved, at stirring, in 96 g of formic acid, and the solution was stirred for 2 hours at 22 °C (room temperature).

Then both solutions were combined and the resulting solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to the maturation bath comprising propane-2-ol, where the coagulation of the fibre was completed. After 120 minutes the maturation bath containing the microfibres was poured into the Eta-Ergo knife mixer, where the fibres were shortened, and further they were shortened and homogenized with the aid of Heidolph DIAX 900 mixer. In the end, the suspension of fibres was filtrated off and thereby the layer of this mixture of fibres was prepared. The obtained filtration cake was washed with methanol and dried at 20 °C.

The bandages based on these fibres are of use for covering smaller acute bleeding wound which tend to dry prematurely.

### Example 19

The procedure was the same as in Example 16. Only the obtained suspension of fibres was diluted 3 times with 2-propanol and the suspension was filtered.

The obtained layer of fibres form hyaluronic acid had the areal weight of 5 g/m² (the weight of the pad was subtracted). The obtained layer of microfibres can be applied including the pad or it can be removed - separated - from the pad and the layer of microfibres can be used separately.

This textile is suitable for flat absorption bandages which stimulate the granulation and wound healing. Combined with an antiseptic agent they can be used for the construction of functional bandages and patches. They can be of use mainly in the treatment of extensive defects. It concerns especially broken surgery wounds and polytraumas.

### Example 20

In the air atmosphere at intense stirring, 1.5 g of sodium hyaluronan (Contipro Biotech s.r.o. Dolní Dobrouč) with the molecular weight of 1.7 MDa (determined by the SEC-MALLS method) were dissolved in 200 g of water to obtain a homogenous, well flowing viscous solution suitable for spinning. 0.257 g of CaCl₂.6 H₂O in 10 ml of water (sample no. 1) or 0.43 g of CaCl₂.6 H₂O in 10 ml of water (sample no. 2) were added to the solution. After thorough mixing, this homogenous solution was spun by the wet method with a nozzle having the diameter of 0.6 mm into the spinning bath comprising propane-2-ol and stirred with the Heidolph DIAX 900 dispergator at 2500 rpm.

The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and further they were shortened and homogenized with the aid of Heidolph DIAX 900 mixer. The suspension of the fibres was further homogenized and in the end filtrated on a suitable pad (knitted fabric from polyamide 6 (silk) with areal weight of approx. 40 g.m⁻²) and thereby a layer of fibres of calcium salt of hyaluronic acid was prepared. The obtained filtration cake was wet compressed in a two-roll calender and dried at 20 °C. The obtained layer of fibres from calcium salt of hyaluronic acid had areal weight of 15 g/m² (the weight of the pad was subtracted). The formed layer of microfibres can be applied including the pad or it can be removed - separated - from the pad and the layer of microfibres can be used separately.

The content of the calcium ion in microfibres was determined by the method of optical emission spectrometry with inductively coupled plasma. The results are shown in the following table:

| Sample | Content of Ca²⁺ (% by weight) |
|---|---|
| 1 | 2.305 |
| 2 | 3.301 |

This textile is suitable as the contact (with the wound) layer for the construction of haemostatic bandages.

### Example 21

In the air atmosphere at intense stirring, 0.5 g of sodium hyaluronan (Contipro Biotech s.r.o. Dolní Dobrouč) with the molecular weight of 1.7 MDa (determined by the SEC-MALLS method) was dissolved in 50 g of water to obtain a homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and then they were shortened and homogenized with the aid of Heidolph DIAX 900 mixer. The suspension of the fibres was further homogenized and in the end filtrated on a suitable pad (knitted fabric from polyamide 6 (silk) with areal weight of approx. 40 g.m⁻²) and thereby a layer of this mixture of fibres was prepared. The obtained filtration cake was wet compressed in a two-roll calender and dried at 20 °C. The obtained layer of fibres from hyaluronic acid had areal weight of 15 g/m² (the weight of the pad was subtracted). The obtained layer of microfibres was inserted without the pad into 100 ml of metallization bath comprising: 65 ml of methanol p.a., 35 ml of formic acid 98% pure, and 1 ml of water, and 2 g of CrF₂ (sample no. 1); or 2 g of KCr(SO₄)₂ · 12H₂O (sample no. 2); or 2 g of CuSO₄ . 5 H₂O (sample no. 3); or 2 g of ZnSO₄ . 7H₂O (sample no. 4); or 2 g of CaCl₂ . 2H₂O (sample no. 5). The layer of microfibres was left in the metallization bath for 25 days, then it was removed, washed with methanol, and dried at room temperature.

The content of the respective cations was determined by the method of optical emission spectrometry with inductively coupled plasma.

The content of the respective metals is shown in the following table.

| sample | Cr % by weight | Cu % by weight | Zn % by weight | Ca % by weight |
|---|---|---|---|---|
| 1 | 3.846 | - | - | - |
| 2 | 2.972 | - | - | - |
| 3 | - | 2.300 | - | - |
| 4 | - | - | 2.806 | - |
| 5 | - | - | - | 0.759 |

The samples nos. 1 and 2 (metallization with chromium ions) were water-insoluble.

The microfibres comprising the metal ions in a farmacologically acceptable concentration are of use in many topical applications. For example the microfibres, or the textile products made of them, comprising calcium ions, can be suitable for the construction of haemostatic bandages. The microfibres, or the textile products made of them, comprising zinc ions or a combination of zinc ions with copper ions, can be utilized as a part of the bandages intended to application on the wounds difficult to heal, as are for example decubitus or venous ulcers. The textile products from the microfibres comprising zinc, copper or chromium ions, or their combination, can be used as a part of covers applied on various forms of pemphigus. The textile products comprising the microfibres bearing zinc ions, or a combination of zinc and copper ions, can be utilized in the combination with various dermatics in preparations suppressing the symptoms of dermatitides. The microfibres comprising zinc or copper ions or their mixtures can be a component of cosmetic

### Example 22

The preparation of the layer from spun HA with the aeral weight of 15 g.m⁻² on a suitable carrier

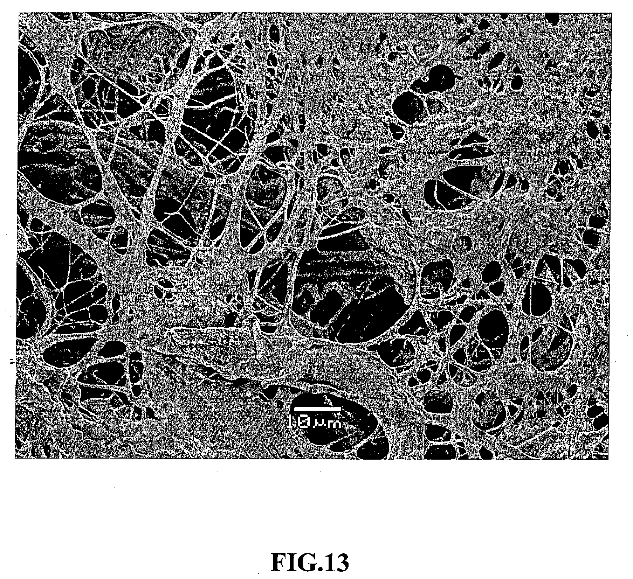

In the air atmosphere at intense stirring, 0.5 g of sodium hyaluronan (CPN s.r.o. Dolní Dobrouč) with the molecular weight of 1.7 MDa (determined by the SEC-MALLS method) was dissolved in 50 g of water to obtain a homogenous, well flowing viscous solution suitable for spinning. This solution was spun by the wet method with a nozzle having the diameter of 0.4 mm into the spinning bath comprising propane-2-ol and stirred with the Heidolph DIAX 900 dispergator at 2500 rpm. The forming microfibre was collected from the bath and transferred to propane-2-ol, where the coagulation of the fibre was completed. After 60 minutes the maturation bath containing the microfibres was poured into the Eta-Ergo knife mixer, where the fibres were shortened, and further they were shortened and homogenized with the aid of Heidolph DIAX 900 mixer. The suspension of the fibres was further homogenized and in the end filtrated on a suitable pad (knitted fabric from polyamide 6 (silk) with areal weight of approx. 40 g.m⁻²) and thereby a layer of this mixture of fibres was prepared. The obtained filtration cake was wet compressed in a two-roll calender and dried at 25 °C and relative air humidity of 65 %. The obtained layer of fibres from hyaluronic acid had the areal weight of 15 g.m⁻² (the weight of the pad was subtracted). The obtained layer of microfibres can be applied including the pad or it can be removed - separated - from the pad and the layer of microfibres can be used separately. The character of the layer is shown in Fig. 13.

The fibres are suitable for flat absorption bandages which stimulate the granulation and wound healing. Combined with an antiseptic agent they can be used for the construction of functional bandages and patches. They can also be useful in the field of plastic and correction surgery.

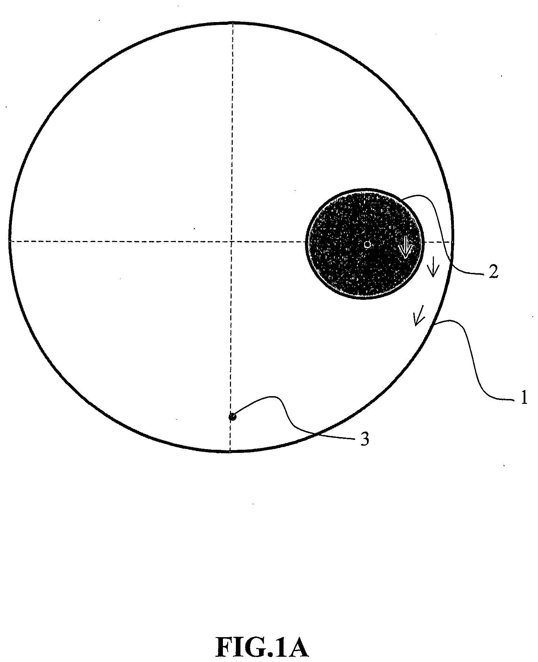

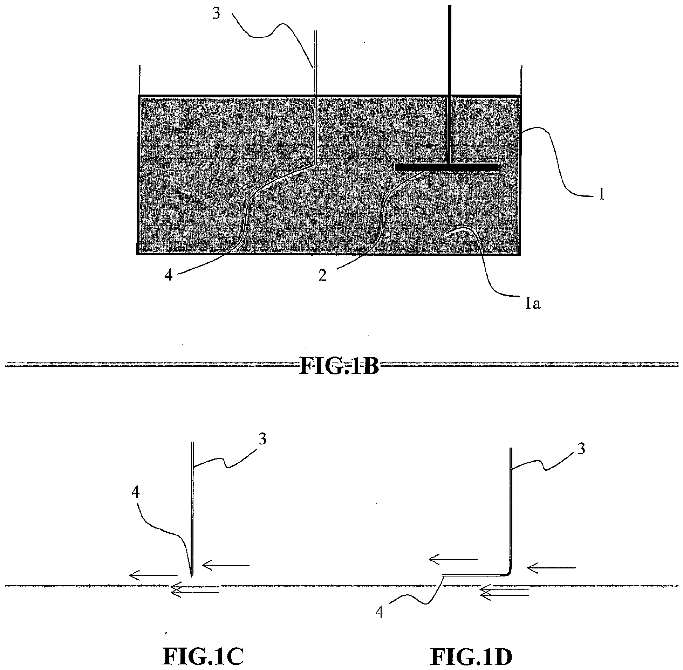

The disclosed examples can be carried out on the apparatus which is shown in Figures 1A and 1B and which comprises a cylindrical container **1** for the coagulation bath **1a,** and a rotary disc **2** with a vertical axis of rotation passing through the bottom of the cylindrical container **1** and in a distance from the vertical axis of the cylindrical container **1**. It means that the rotary disc **2** (rotor) which causes the movement of the coagulation bath, is placed asymmetrically in the cylindrical container **1**. The diameter of the rotary disc **2** is approximately 10 to 30 % of the diameter of the container **1**. The distance of the edge of rotary disc **2** from the wall of the container **1** is in the case of laboratory apparatus approximately 5 to 20 mm, for example 15 mm. The advantages of this arrangement are that the formed fibres are not wound up on the rotary disc **2,** they do not break, and they are not twisted mutually. The rotary disc **2** is preferably made of steal, glass or ceramics of the thickness of 1 to 3 mm, and the diameter can be for example 60 mm, and it is placed in the coagulation bath. The coagulation bath can be of the volume for example 2000 cm³, and is placed in the container **1** of a cylindrical character with the diameter of 230 mm.

The rotations of the rotary disc **2** are preferably 2000 to 2500 rpm. The arrows in Figs. 1A and 1C and 1D show the direction of the disc rotation, or the direction of the stream of the coagulation bath.

The apparatus further comprises the spinning nozzle **3,** the outlet **4** of which is arranged in the cylindrical container **1** in a distance from its wall of 70 to 130 % of the shortest distance of the rotary disc **2** form the wall of the cylindrical container **1**. The opening of the nozzle **3** (needle) bringing the polymer solution has preferably the diameter of 0,05 to 1,6 mm and is placed in the direction of the stream of the coagulation bath in the distance of 80 to 190° from the axis passing through the centre of the container **1** and the centre of the rotary disc **2** (Fig. 1A). It means that the angle spacing of the vertical axis of the rotary disc **2** and the outlet **4** of the spinning nozzle **3** is preferably above 80°, related to the vertical axis of the container **1.** In another preferred embodiment of the invention the spinning nozzle **3** can be bent on its end comprising the outlet **4.**

The advantage of the bending of the nozzle **3** is that the axis of the stream leaving the nozzle **3** is concurrent with the stream of the coagulation bath and that the bending of the nozzle **3,** due to the deformation and narrowing of the diameter in the point of bending, causes a stronger "stress" to the macromolecules of the polymer, and therefore, for example due to the thixiotropic character exhibited by hyaluronan, the kinetic viscosity of the polymer solution is decreased and also a "linear arrangement of the chain of macromolecules" occurs which is a good condition for the formation of tight fibres. According to the method of spinning, the outlet **4** of the nozzle **3** can be placed above or under the level of the coagulation bath **1a.**

## Claims

1. A method of preparation of polysaccharide staple microfibres for wound covers **characterized in that** a 0.01 to 8 %wt spinning solution of a polysaccharide having the molecular weight of 60 kDa to 3 MDa and being selected from the group comprising hyaluronic acid, a compound comprising hyaluronic acid and metal ions, schizophylan, chitin/chitosan-glucan complex, a compound comprising chitin/chitosan-glucan complex and metal ions, internal mixture of chitin and chitosan, a compound comprising internal mixture of chitin and metal ions, sodium alginate, potassium alginate, ammonium alginate, xanthane, xanthane sodium salt, xanthane potassium salt, oxycellulose, oxycellulose sodium salt, oxycellulose potassium salt, or mixtures of said
polysaccharides, is prepared in an aqueous medium, which is
spun in a non-stationary coagulation bath consisting of 97.5 to 100 % C₁-C₃ alcohol, wherein the coagulation bath is stirred at 2,000 to 2,500 rpm,
the resulting fibres being further transferred
for 20 minutes to 96 hours from the coagulation bath to the stationary maturation bath comprising C₁-C₃ alcohol, and then dried, or washed with alcohol and then dried.

2. The method of preparation according to claim 1 **characterized in that** the spinning solution passes through an air passage of the length of 1 to 200 mm before its entering the coagulation bath, and/or the spinning solution, before entering the coagulation bath, is stored in a refrigerator for 5 to 24 hours at the temperature of -40 °C to -10°C and then it is left to heat to room temperature.

3. The method of preparation according to any one of claims 1 to 2 **characterized in that** the temperature of the coagulation bath is within the range of 18 to 30 °C.

4. The method of preparation according to any of the preceding claims **characterized in that** the maturation bath further comprises up to 10 % by weight of organic acid.

5. The method of preparation according to any of the preceding claims **characterized in that** the spinning solution further comprises a calcium or zinc salt in the concentration of 0.1 to 50 % by weight.

6. The method of preparation according to any of the preceding claims **characterized in that** the fibres are further metallized for 20 minutes to 25 days directly in bath selected from the coagulation bath, maturation bath, shortening bath or homogenization bath, by adding a salt of polyvalent metal, or they are transferred to a new bath, wherein each of the baths comprises C₁-C₃ alcohol, or a mixture of C₁-C₃ alcohol and organic acid, and a salt of polyvalent metal selected from the group comprising calcium, magnesium, copper, manganese, zinc, chromium, iron, and cobalt.

7. The method of preparation according to any of the preceding claims **characterized in that** the fibres are shortened in the shortening bath comprising C₁-C₃ alcohol, or the mixture of C₁-C₃ alcohol and organic acid, from the starting length of 0.8 to 3 cm to the length of 1 to 5 mm, or the fibres are shortened directly in the maturation bath, or they are transferred to a new shortening bath.

8. The method of preparation according to any of the preceding claims **characterized in that** the fibres are homogenized in the homogenization bath comprising C₁-C₃ alcohol, or the mixture of C₁-C₃ alcohol and organic acid, or they are homogenized directly in the shortening bath, or they are transferred to a new homogenization bath.

9. A method of production of a wound
cover based on polysaccharide fibres **characterized in that** the
fibres are prepared by the method defined in any of claims 1 to 8, or a mixture of fibres prepared by the method defined in any of claims 1 to 8 is prepared, or a mixture of fibres prepared by the method defined in any of claims 1 to 8 and fibres based on the natural mycelium is prepared, and then the fibres are filtrated off the solution and the filtration cake is compressed and/ or dried.

10. The method of production of the cover according to claim 9 **characterized in that** the fibres are filtrated onto a suitable pad, the filtration cake is compressed with the pad, and then they are dried, wherein the filtration cake is optionally washed with alcohol before drying and/or the pad is optionally removed from the dried filtration cake.

11. The method of production of the cover according to any of claims 9 or 11 **characterized in that** the dried filtration cake is inserted into the metallization bath, then the fibres are removed from the metallization bath, washed with alcohol and dried.

12. The method of production of the cover according to any of claims 9 to 11 **characterized in that** the drying is carried out at the temperature of 20 to 80 °C and relative air humidity of 15 to 80 %.

13. The method of production of the cover according to any of claims 9 to 12 **characterized in that** the drying is carried out by hot air.

14. A wound cover based on the polysaccharide fibres prepared by the method defined in any of claims 1 to 8 **characterized in that** it comprises staple microfibres of biopolymer, or a mixture of staple microfibres of biopolymer, or a mixture of staple microfibres of biopolymer and fibres based on the natural mycelium, where its areal weight is at least 5 g/m².

15. The wound cover according to claim 14 **characterized in that** it is in the form of nonwoven textile, nonwoven textile fixed on a pad, cotton wool, tampon or paper stuff.

## Patentansprüche

1. Verfahren zur Vorbereitung von polysaccharidischen Stapel-Mikrofasern für Wunddeckungen, **dadurch gekennzeichnet, dass** eine zu verspinnende Lösung, deren Konzentration 0,01 bis 8 Gew.-% beträgt, eines Polysaccharides, dessen Molekulargewicht im Bereich von 60 kDa bis 3 MDa liegt und das aus der Gruppe umfassend die Hyaluronsäure, eine die Hyaluronsäure und Metallionen enthaltende Verbindung, einen Schizophylan, einen Chitin/Chitosan-Glucan-Komplex, eine einen Chitin/Chitosan-Glucan-Komplex und Metallionen enthaltende Verbindung, ein inneres Gemisch von Chitin und Chitosan, eine einen inneren Gemisch von Chitin und Chitosan und Metallionen enthaltende Verbindung, Natriumalginat, Kaliumalginat, Ammoniumalginat, Xanthan, Xanthan-Natriumalginat, Xanthan Kaliumalginat, Oxycellulose, Oxycellulose-Natriumsalz, Oxycellulose-Kaliumsalz, oder Gemische der besagten Polysaccharide ausgewählt wird, in einem wässrigen Medium vorbereitet wird und in einem nicht stationären Koagulierbad, das aus einem C₁-C₃-Alkohol mit einem Mengenanteil im Bereich von 97,5 bis 100 % besteht, versponnen wird, wobei das Koagulierbad mit einer Geschwindigkeit im Bereich von 2 000 bis 2 500 U/min umgerührt wird, wobei die derart hergestellten Fasern ferner aus dem Koagulierbad in ein einen C₁-C₃-Alkohol enthaltendes stationäres Reifungsbad übertragen werden, wo sie 20 Minuten bis 96 Stunden lang verweilen, und anschliessend entweder getrocknet oder mit Alkohol gewaschen und daraufhin getrocknet werden.

2. Verfahren zur Vorbereitung nach Anspruch 1, **dadurch gekennzeichnet, dass** die zu verspinnende Lösung durch einen Luftkanal mit der Länge von 1 bis 200 mm geführt wird, bevor sie in das Koagulierbad gelangt, und/oder die zu verspinnende Lösung 5 bis 24 Stunden lang in einem Kühlschrank bei einer Temperatur im Bereich von -40 °C bis -10 °C aufbewahrt wird, bevor sie in das Koagulierbad gelangt, und anschliessend sie erwärmt wird, um die Raumtemperatur zu erreichen.

3. Verfahren zur Vorbereitung nach einem der Ansprüche 1 oder 2, **dadurch gekennzeichnet, dass** die Temperatur des Koagulierbad im Bereich von 18 bis 30 °C liegt.

4. Verfahren zur Vorbereitung nach einem der vorhergehenden Ansprüche, **dadurch gekennzeichnet, dass** das Reifungsbad ferner eine organische Säure umfasst, deren Gewichtsanteil bis 10 % beträgt.

5. Verfahren zur Vorbereitung nach einem der vorhergehenden Ansprüche, **dadurch gekennzeichnet, dass** die zu verspinnende Lösung ferner ein Kalzium- oder Zinksalz umfasst, dessen Konzentration 0,1 bis 50 % Gew.-% beträgt.

6. Verfahren zur Vorbereitung nach einem der vorhergehenden Ansprüche, **dadurch gekennzeichnet, dass** die Fasern ferner 20 Minuten bis 25 Tage lang direkt in einem Bad metallisiert werden, das von der Gruppe umfassend das Koagulierbad, das Reifungsbad, das Verkürzungsbad oder das Homogenisierungsbad ausgewählt wird, und zwar durch die Zugabe eines Salzes eines mehrwertigen Metalls, oder dass die Fasern in ein neues Bad eingebracht werden, wobei jedes der Bäder einen C₁-C₃-Alkohol oder ein Gemisch eines C₁-C₃-Alkohols und einer organischen Säure gemeinsam mit einem Salz eines mehrwertigen Metalls umfasst, welches Salz aus der Gruppe ausgewählt wird, die Kalzium, Magnesium, Kupfer, Mangan, Zink, Chrom, Eisen und Kobalt umfasst.

7. Verfahren zur Vorbereitung nach einem der vorhergehenden Ansprüche, **dadurch gekennzeichnet, dass** die Fasern in dem Verkürzungsbad verkürzt werden, das einen C₁-C₃-Alkohol oder ein Gemisch eines C₁-C₃-Alkohols umfasst, wobei die anfängliche Länge 0,8 bis 3 cm und die zu erreichende Länge 1 bis 5 mm beträgt, oder dass die Fasern direkt in dem Reifungsbad verkürzt werden, oder dass die Fasern in ein neues Verkürzungsbad übertragen werden.

8. Verfahren zur Vorbereitung nach einem der vorhergehenden Ansprüche, **dadurch gekennzeichnet, dass** die Fasern in dem Homogenisierungsbad homogenisiert werden, das einen C₁-C₃-Alkohol oder ein Gemisch eines C₁-C₃-Alkohols umfasst, oder dass die Fasern direkt in dem Verkürzungsbad homogenisiert werden, oder dass die Fasern in ein neues Homogenisierungsbad übertragen werden.

9. Verfahren zur Herstellung einer Wunddeckung auf der Basis von polysaccharidischen Fasern, **dadurch gekennzeichnet, dass** die Fasern in dem Verfahren nach einem der Ansprüche 1 bis 8 vorbereitet werden, oder das ein Gemisch von Fasern vorbereitet wird, die in dem Verfahren nach einem der Ansprüche 1 bis 8 vorbereitet wurden, oder das ein Gemisch von in dem Verfahren nach einem der der Ansprüche 1 bis 8 zuvor vorbereiteten Fasern mit Fasern auf der Basis eines natürlichen Myzeliums vorbereitet wird, und anschliessend die derart vorbereiten Fasern aus der Lösung ausgefiltert werden und der Filterkuchen gepresst und/oder ausgetrocknet wird.

10. Verfahren zur Herstellung einer Wunddeckung nach Anspruch 9, **dadurch gekennzeichnet, dass** die Fasern auf einer geeigneten Unterlage gefiltert werden, wobei der Filterkuchen mit der Unterlage gepresst wird, und anschliessend die Fasern getrocknet werden, wobei der Filterkuchen wahlweise vor der Austrocknung mit einem Alkohol gewaschen wird und/oder die Unterlage wahlweise von dem ausgetrocknetem Filterkuchen abgetrennt wird.

11. Verfahren zur Herstellung einer Wunddeckung nach einem der Ansprüche 9 oder 10, **dadurch gekennzeichnet, dass** der ausgetrocknete Filterkuchen in ein Metallisierbad eingebracht wird und dass anschliessend die aus dem Metallisierbad herausgenommenen Fasern mit einem Alkohol gewaschen werden und daraufhin getrocknet werden.

12. Verfahren zur Herstellung einer Wunddeckung nach einem der Ansprüche 9 bis 11, **dadurch gekennzeichnet, dass** die Austrocknung bei einer Temperatur im Bereich von 20 bis 80 °C und bei einer relativen Luftfeuchtigkeit im Bereich von 15 bis 80 % erfolgt.

13. Verfahren zur Herstellung einer Wunddeckung nach einem der Ansprüche 9 bis 12, **dadurch gekennzeichnet, dass** die Austrocknung mit heißer Luft durchgeführt wird.

14. Wunddeckung auf der Basis von polysaccharidischen Fasern, vorbereitet in dem Verfahren nach einem der Ansprüche 1 bis 8, **dadurch gekennzeichnet, dass** sie Stapel-Mikrofasern aus einem Biopolymer oder ein Gemisch von Stapel-Mikrofasern aus Biopolymeren oder ein Gemisch von Stapel-Mikrofasern aus Biopolymeren mit Fasern auf der Basis eines natürlichen Myzeliums umfasst, wobei ihr Flächengewicht mindestens 5 g/m² beträgt.

15. Wunddeckung nach Anspruch 14, **dadurch gekennzeichnet, dass** sie in der Form einer ungewebten Textilie, einer auf einer Unterlage angebrachten ungewebten Textilie, einer Watte, eines Tampons oder einer Papiermasse vorliegt.

## Revendications

1. Un procédé de préparation de microfibres discontinues de polysaccharide pour des couvertures de plaies, **caractérisé en ce qu'**une solution de filage de 0,01 à 8 pourcentage de poids d'un polysaccharide ayant le poids moléculaire de 60 kDa à 3 MDa et étant choisi dans le groupe comprenant l'acide hyaluronique, un composé de l'acide hyaluronique et des ions métalliques, schizophylane, un complexe de chitine / chitosanglucane, un composé comprenant le complexe de chitine / chitosane-glucane et des ions métalliques, un mélange interne de chitine et de chitosane, un composé comprenant un mélange interne de chitine et d'ions métalliques, alginate de sodium, alginate de potassium , alginate d'ammonium, xanthane, un sel de sodium de xanthane, un sel de potassium de xanthane, oxycellulose, un sel de sodium d' oxycellulose, un sel de potassium d'oxycellulose, ou des mélanges de ces polysaccharides, est préparé dans un milieu aqueux, est filé dans un bain de coagulation non stationnaire se composant de 97,5 à 100% d'alcool de C₁-C₃, où le bain de coagulation est agité entre 2000 et 2500 tr / min, les fibres résultantes étant ensuite transférées pour 20 minutes à 96 heures du bain de coagulation au bain de maturation stationnaire comprenant l'alcool en C₁-C₃, et puis séchées, ou lavées à l'alcool et puis séchées.

2. Le procédé de préparation selon la revendication 1 **caractérisé en ce que** la solution de filage traverse un passage de l'air de 1 à 200 mm de longueur avant son entrée dans le bain de coagulation, et / ou la solution de filage, avant d'entrer dans le bain de coagulation, est déposée dans un réfrigérateur pendant 5 à 24 heures à une température de -40 ° C à -10 ° C, et puis elle est laissée chauffer à température ambiante.

3. Le procédé de préparation selon l'une quelconque des revendications 1 à 2 **caractérisé en ce que** la température du bain de coagulation est comprise entre 18 ° C et 30 °C.

4. Le procédé de préparation selon l'une quelconque des revendications précédentes, **caractérisé en ce que** le bain de maturation comprend en outre jusqu'à 10% en poids d'acide organique.

5. Le procédé de préparation selon l'une quelconque des revendications précédentes, **caractérisé en ce que** la solution de filage comprend en outre un sel de calcium ou de zinc à la concentration de 0,1 à 50% en poids.

6. Le procédé de préparation selon l'une quelconque des revendications précédentes **caractérisé en ce que** les fibres sont en outre métallisées pendant 20 minutes à 25 jours directement dans le bain choisi parmi les bains de coagulation, bain de maturation, bain de accourcissant ou bain d'homogénéisation, par addition d'un sel d'un métal polyvalent, ou elles sont transférées dans un nouveau bain, où chacun des bains comprend un alcool C₁-C₃ ou un mélange d'alcool C₁-C₃, et d'acide organique, et un sel d'un métal polyvalent choisi dans le groupe comprenant le calcium, le magnésium, le cuivre, manganèse, zinc, chrome, fer et cobalt.

7. Le procédé de préparation selon l'une quelconque des revendications précédentes **caractérisé en ce que** les fibres sont accourcies dans le bain accourcissant comprenant de l'alcool C₁-C₃, ou le mélange d'alcool C₁-C₃, et d'acide organique, à partir de la longueur de départ de 0,8 à 3 cm à la longueur de 1 à 5 mm, ou les fibres sont accourcies directement dans le bain de maturation, ou elles sont transférées dans un nouveau bain accourcissant.

8. Le procédé de préparation selon l'une quelconque des revendications précédentes **caractérisé en ce que** les fibres sont homogénéisées dans le bain d'homogénéisation comprenant de l'alcool en C₁-C₃, ou un mélange d'alcool en C₁-C₃ et d'acide organique, ou bien elles sont homogénéisées directement dans le bain accourcissant, ou elles sont transférées dans un nouveau bain d'homogénéisation.

9. Un procédé de fabrication de la couverture de plaie à base de fibres de polysaccharide **caractérisé en ce que** les fibres sont préparées par le procédé défini dans l'une quelconque des revendications 1 à 8, ou un mélange de fibres préparées par le procédé défini dans l'une quelconque des revendications 1 à 8 est préparé, ou un mélange de fibres préparées par le procédé défini dans l'une quelconque des revendications 1 à 8 et des fibres à base de mycélium naturel est préparé, et puis les fibres sont filtrées de la solution et la galette de masse filtrante est comprimée et / ou séchée.

10. Le procédé de fabrication de la couverture selon la revendication 9 **caractérisé en ce que** les fibres sont filtrées sur un support approprié, la galette de masse filtrante est comprimée avec le support, puis ils sont séchés, la galette de masse filtrante étant éventuellement lavée à l'alcool avant le séchage et / ou le support étant éventuellement retiré de la galette de masse filtrante séchée.

11. Le procédé de fabrication de la couverture selon l'une quelconque des revendications 9 ou 10 **caractérisé en ce que** la galette de masse filtrante séchée est insérée dans le bain de métallisation, puis les fibres sont retirées du bain de métallisation, lavées à l'alcool et séchées.

12. Le procédé de fabrication de la couverture selon l'une quelconque des revendications 9 à 11 **caractérisé en ce que** le séchage est effectué à la température de 20 ° C à 80 ° C et à l'humidité relative de l'air de 15 à 80%.

13. Le procédé de fabrication de la couverture selon l'une quelconque des revendications 9 à 12 **caractérisé en ce que** le séchage est effectué par de l'air chaud.

14. Une couverture des plaies à base de fibres polysaccharidiques préparées par le procédé défini dans l'une quelconque des revendications 1 à 8, **caractérisé en ce qu'**il comprend des microfibres discontinues de biopolymère, ou un mélange de microfibres discontinues de biopolymère, ou un mélange de microfibres discontinues de biopolymère et fibres à base de mycélium naturel, dont le poids de surface est d'au moins 5 g / m².

15. La couverture des plaies selon la revendication 14 **caractérisé en ce qu'**il se présente sous la forme de textile non-tissé, de textile non-tissé fixé sur un support, d'ouate, de tampon ou de pâte de papier.s
